(19) Europäisches Patentamt
European Patent Office
Office européen des brevets

(11) Publication number: **0 083 204**
**A2**

# (12) EUROPEAN PATENT APPLICATION

(21) Application number: 82306868.9

(22) Date of filing: 22.12.82

(51) Int. Cl.³: C 07 C 91/44
C 07 C 103/82, C 07 C 147/14
C 07 C 149/42, C 07 D 277/66
A 61 K 31/135, A 61 K 31/165
A 61 K 31/425

(30) Priority: 28.12.81 JP 209740/81

(43) Date of publication of application:
06.07.83 Bulletin 83/27

(84) Designated Contracting States:
AT BE CH DE FR GB IT LI LU NL SE

(71) Applicant: ONO PHARMACEUTICAL CO., LTD.
No. 14, Doshomachi 2-chome Higashi-ku
Osaka-shi Osaka(JP)

(72) Inventor: Wakatsuka, Hirohisa
No 3-3-708, Miyano-cho
Takatsuki-shi Osaka(JP)

(72) Inventor: Mohri, Tetsuya
14-3 Ohsumigaoka 4-chome
Tanabe-cho Tsuzuki-gun Kyoto(JP)

(72) Inventor: Hashimoto, Shinsuke
No 2-6-905 Shirakawa 3-chome
Ibaraki-shi Osaka(JP)

(74) Representative: Pearce, Anthony Richmond et al,
Marks & Clerk Alpha Tower Suffolk Street Queensway
Birmingham B1 1TT(GB)

## (54) 2-Aminophenol derivatives and process for their preparation.

(57) 2-AMINOPHENOL derivatives of the general formula:

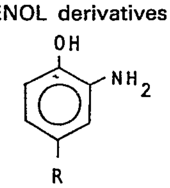

(I)

[wherein R represents a group of the general formula:

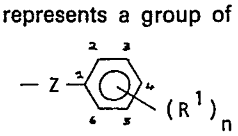

(IIa)

(in which Z represents a sulfur atom or a sulfinyl group and $(R^1)_n$ represents that:
(i) n is an integer of 1, 2 or 3 and $R^1$, which may be the same or different, is a hydrogen atom, a straight- or branched-chain alkyl group containing from 1 to 6 carbon atoms or a halogen atom;
(ii) n is an integer of 1, 2 or 3 and $R^1$ is a hydroxy group; or
(iii) n is an integer of 2 and two $R^1$s are a hydroxy group and an amino group, a hydroxy group and a straight- or branched-chain alkyl group containing from 1 to 6 carbon atoms, or a hydroxy group and a halogen atom, such groups being at o-position or p-position to each other), or
R represents a group of the general formula:

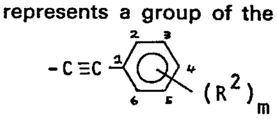

(IIb)

(in which m is an integer of 1 or 2 and $R^2$, which may be the same or different, represents a hydrogen atom, a straight- or branched-chain alkyl group containing from 1 to 6 carbon atoms or a halogen atom), or R represents a group of the general formula:

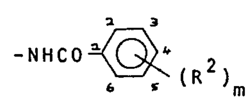

(IIc)

(in which the various symbols are as hereinbefore defined), or R represents a group of the general formula:

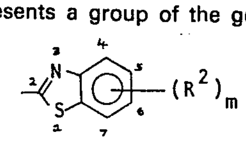

(IId)

./...

EP 0 083 204 A2

# S P E C I F I C A T I O N

## 2-AMINOPHENOL DERIVATIVES AND PROCESS FOR THEIR PREPARATION

This invention is concerned to new 2-aminophenol derivatives which have an inhibitory activity on 5-lipoxygenase specifically or an inhibitory activity on 5-lipoxygenase and cyclooxygenase at the same time, the process for their preparation and pharmaceutical compositions containing them as an active ingredient.

Up to now, several compounds which inhibit lipoxygenase have been known, that is to say, 5,8,11,14-eicosatetraynoic acid (i.e. ETYA), 5,8,11-eicosatriynoic acid, acetone phenylhydrazone (i.e. APH), phenidone (i.e. 1-phenyl-3-pyrazolidone), NDGA (nordihydroguaiaretic acid), BW-775C (3-amino-1-p-trifluoromethyl-pyrazole), 3-amino-1-p-chlorophenyl-2-pyrazoline, benoxaprofene.

These above compounds inhibit all lipoxygenases and other enzymes in the arachidonate cascade at the same time, or inhibit all lipoxygenases of 5-, 12- and 15-lipoxygenase [see Gendai Iryo , 12, 1065 (1980)].

The compounds of the present invention are particularly superior in having selective inhibitory activity on 5-lipoxygenase of many lipoxygenases,which react at the first stage of producing various kinds of leukotrienes from arachidonic acid in a living body and further not inhibiting other enzymes in the arachidonate cascade, or in having inhibitory activity on 5-lipoxygenase and cyclooxygenase which reacts at the first step of producing prostaglandins from arachidonic acid similarly, at the same time.

This invention is concerned to new 2-aminophenol derivatives which have an inhibitory activity on 5-lipoxygenase, or an inhibitory activity on 5-lipoxygenase and on cyclooxygenase at the same time, the process for their preparation and pharmaceutical compositions containing them as an active ingredient. More particularly, there are a group of compounds called leukotrienes, which are biosynthesized from arachidonic acid by a series of reactions in a living body and 5-lipoxygenase is related to the first step of this reaction. Similarly, prostaglandins are biosynthesized from arachidonic acid by another series of reactions, and cyclooxygenase is related to the first step of such reaction. This invention is concerned to new 2-aminophenol derivatives which strongly inhibit 5-lipoxygenase or strongly inhibit 5-lipoxygenase and cyclooxygenase at the same time, and which are, therefore, useful as treating and preventing agents for diseases which induced by leukotrienes, e.g. allergic tracheal and bronchial diseases or allergic lung diseases, allergic shocks or various allergic inflammations, and various inflammations induced by prostaglandins, and further, this invention is concerned to the process for their preparation and pharmaceutical compositions comprising them as an active ingredient.

In the study of prostaglandins (abbreviated as PG hereafter), many important discoveries have been made continuously in recent years. And so it was found a large change in the direction of the research and development of PG. In the compounds which have been newly found or newly confirmed their structure in PG family, it can be said that PG endoperoxides, (i.e. $PGG_2$ and $PGH_2$), thromboxane $A_2$ (abbreviated as $TXA_2$ hereafter), prostacyclin (i.e. $PGI_2$) and leukotriene C, D and E

(abbreviated as LTC, LTD and LTE, respectively, hereafter) etc. have especially strong and unique biological activities.

All the compounds of PG family containing various PG previously known well in addition to the above compounds, are biosynthesized from the same mother compound, i.e. arachidonic acid in a living body and, therefore, the metabolic routes starting from arachidonic acid is called "Arachidonate cascade" as a whole. The detailed explanation of each route and the pharmacological character of each metabolite are described in Igaku no Ayumi, 114, 378 (1980), ibid, 114, 462 (1980), ibid, 114, 866 (1980), ibid, 114, 929 (1980), Gendai Iryo, 12, 909 (1980), ibid, 12, 1029 (1980), ibid, 12, 1065 (1980) and ibid, 12, 1105 (1980) etc.

The arachidonate cascade can be largely divided into two routes; one is the route that cyclooxygenase acts on arachidonic acid to convert, via $PGG_2$ and further $PGH_2$, into various PG, e.g. prostaglandin $F_{2}\alpha$ (abbreviated $PGF_{2}\alpha$ hereafter), prostaglandin $E_2$ (abbreviated $PGE_2$ hereafter), $PGI_2$, $TXA_2$, and the other is the route that lipoxygenase acts on arachidonic acid to convert, via hydroperoxyeicosatetraenoic acid (abbreviated HPETE hereafter), into hydroxyeicosatetraenoic acid (abbreviated as HETE hereafter) or leukotrienes.

As the former route is well known, it is not described in the present specification in detail. See Prostaglandin (1978), edited by Makoto Katori, published by Kohdan-sha.

Concerning the latter route, it has been known that various compounds are produced according to the following Scheme A.

SCHEME A

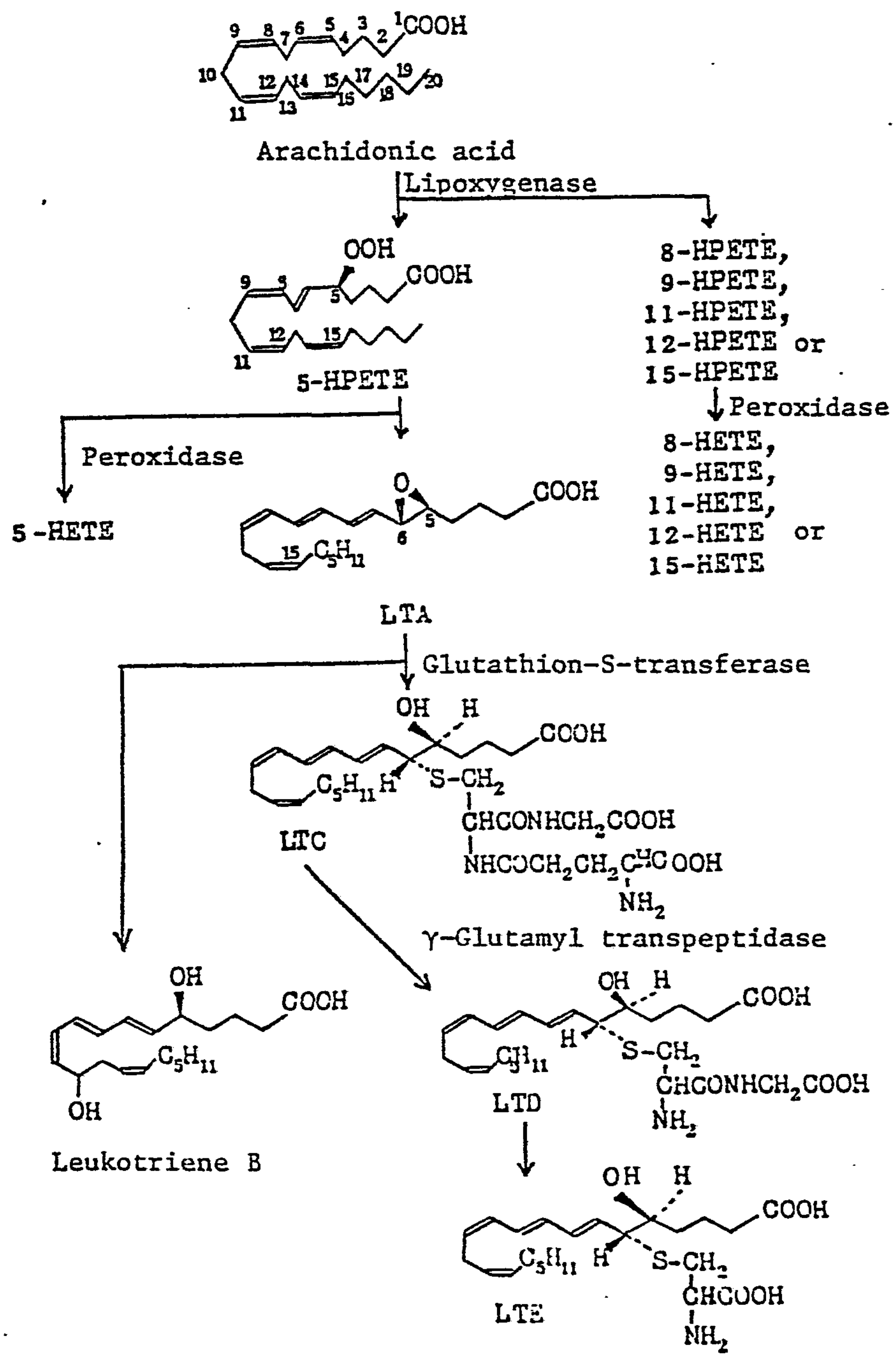
Arachidonic acid
Lipoxygenase
5-HPETE
8-HPETE,
9-HPETE,
11-HPETE,
12-HPETE or
15-HPETE
Peroxidase
8-HETE,
9-HETE,
11-HETE,
12-HETE or
15-HETE
Peroxidase
5-HETE
LTA
Glutathion-S-transferase
LTC
γ-Glutamyl transpeptidase
Leukotriene B
LTD
LTE

Besides being metabolized through a well known route, i.e. the route via PG endoperoxides, arachidonic acid is also metabolized through another route by the action of lipoxygenase. That is to say, arachidonic acid is metabolized by the action of lipoxygenase, e.g. 5-lipoxygenase, 12-lipoxygenase and 15-lipoxygenase, to 5-HPETE, 12-HPETE and 15-HPETE, respectively, having following formulae:

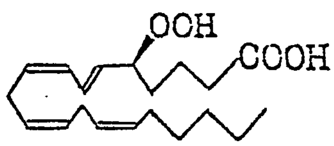

5-HPETE

,

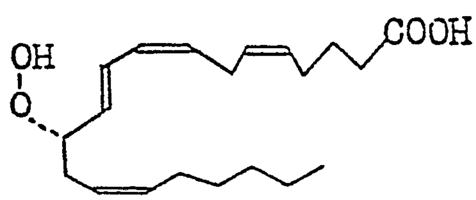

12-HPETE

and

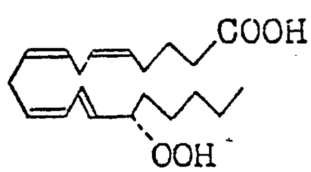

15-HPETE

These HPETE are converted into 5-HETE, 12-HETE and 15-HETE, respectively, by the action of peroxidase converting a hydroperoxy group into a hydroxy group. Furthermore, LTA is also produced from 5-HPETE. LTA is converted into leukotriene B (abbreviated as LTB hereafter) enzymatically or not enzymatically, or is converted into LTC by the action of glutathion-S-transferase. Further, LTC is converted into LTD by the action of γ-glutamyl transpeptidase.

Moreover, it was recently defined that LTD is metabolized to LTE [see Biochem. Biophys. Res. Commun., 91, 1266 (1979) and Prostaglandins, 19(5), 645 (1980)].

It was found that LTC and LTD are identical with SRS (slow reacting substance) or SRS-A (slow reacting substance of anaphylaxis) which was well known before [see Proc. Natl. Acad. Sci. USA, 76, 4275 (1979), Biochem. Biophys. Res. Commun., 91, 1266 (1979), Proc. Natl. Acad. Sci. USA, 77, 2014 (1980) and Nature, 285, 104 (1980)]. So it can be understood that the pharmacological characters of these leukotrienes are the same as those of SRS or SRS-A.

Feldberg et al. reported that a substance is released when perfusing cobra venom through isolated lung or incubating cobra venom with vitellus. The substance was named SRS and it has been found that SRS constricts ilem isolated from guinea pig slowly and continually [see J. Physiol., 94, 187 (1938)].

Moreover, Kellaway et al. showed the relation between SRS-A and allergic reaction from the fact that SRS-A is released when an antigen is sensitized to perfusing lung isolated from guinea pig [see Quant. J. Exp. Physiol., 30, 121 (1940)].

Brocklehurst reported that when the antigen is sensitized to a

lung fragment isolated from a bronchial asthmatics whose specific antigen is defined, by an operation, histamine and SRS-A are released and strongly constrict bronchial muscle. Since such constriction can not be prevented by an antihistaminic agent, he suggested that SRS-A is an important bronchoconstrictor in an asthmatic paroxysm [see Progr. Allergy, 6, 539 (1962)].

Since then, many reports were published, for instance, SRS-A prepared from human lung slice constrict a tracheal spiral of normal human [see Int. Arch. Allergy Appl. Immunol., 38, 217 (1970)]; when SRS-A prepared from rats is injected intravenously to guinea pig, significant increase of purlmonory resistance is observed [see J. Clin. Invest., 53, 1679 (1974)]; in addition, a subcutaneous injection of SRS-A to guinea pig, rat and monkey makes vascular permeability to enhance. [see Advances in Immunology, 10, 105 (1969), J. Allergy Clin. Immunol., 621, 371 (1978), Prostaglandins, 19(5), 779 (1980) etc.].

Generally, the substance released by immunological reaction is called SRS-A. On the other hand, the substance released by non-immunological reaction such as calcium ionophore is called SRS. However, the above two substances have many similarities each other and, therefore, it is considered they would probably be the same substance.

Based on result of these studies, various leukotrienes (LTC, LTD and LTE, and further other leukotrienes which may be confirmed their structures in future) biosynthesized from arachidonic acid via LTA, are considered to be important factors relating to the appearance of allergic tracheal and bronchial disease, allergic lung disease, allergic shock and various allergic inflammations.

Recently, it was proposed that PG is an important chemical

mediator of inflammatory reaction. For example, it was reported that PGE enhances a vascular permeability and a pain, and has a vasodilative action, pyrogenetic action and chemotactic action [see Prostaglandin (1978), edited by Makoto Katori, published by Kohdan-sha]. Furthermore, it was reported that $PGI_2$ alone does not affect vascular permeability, but it enhances vascular permeability of histamine [see Prostaglandins, 15, 557 (1978)]. Moreover, the most recent report indicated that in order to suppress such action of PG, it is more effective to inhibit not only 5-lipoxygenase but also cyclooxygenase at the same time [see Biochemical Pharmacology, 28, 1959 (1979) and European Journal of Pharmacology, 66, 81 (1980)].

As the result of energetic investigations in order to find new compounds inhibiting 5-lipoxygenase, or inhibiting 5-lipoxygenase and cyclooxygenase at the same time in a living body, and so being effective for prevention and treatment of not only various allergic diseases induced by leukotrienes but also inflammations induced by PGs, we have found the compounds which can be achieved that purpose, having completed this invention.

The present invention accordingly provides new 2-aminophenol derivatives of the general formula:

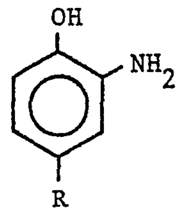

(I)

[wherein R represents a group of the general formula:

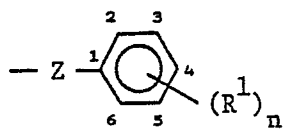

(IIa)

(in which Z represents a sulfur atom or a sulfinyl group and $(R^1)_n$ represents that:

(i) n is an integer of 1, 2 or 3 and $R^1$, which may be the same or different, is a hydrogen atom, a straight- or branched-chain alkyl group containing from 1 to 6 carbon atoms or a halogen atom;

(ii) n is an integer of 1, 2 or 3 and $R^1$ is a hydroxy group; or

(iii) n is an integer of 2 and two $R^1$s are a hydroxy group and an amino group, a hydroxy group and a straight- or branched-chain alkyl group containing from 1 to 6 carbon atoms, or a hydroxy group and a halogen atom, such groups being at o-position or p-position to each other), or

R represents a group of the general formula:

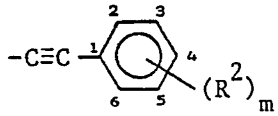

(IIb)

(in which m is an integer of 1 or 2 and $R^2$, which may be the same or different, represents a hydrogen atom, a straight- or branched-chain alkyl group containing from 1 to 6 carbon atoms or a halogen atom), or R represents a group of the general formula:

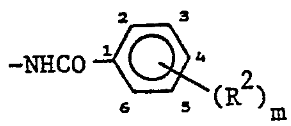

(IIc)

(in which the various symbols are as hereinbefore defined), or R represents a group of the general formula:

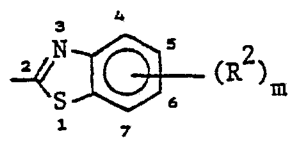

(IId)

(in which the various symbols are as hereinbefore defined)], and pharmaceutically-acceptable acid addition salts thereof.

In the general formulae (IIa), (IIb), (IIc) and (IId), examples of the straight- or branched-chain alkyl group containing from 1 to 6 carbon atoms represented by $R^1$ and $R^2$, are methyl, ethyl, propyl, butyl, pentyl and hexyl and their isomers, preferably methyl or tert-butyl.

In the general formulae (IIa), (IIb), (IIc) and (IId), examples of the halogen atom represented by $R^1$ and $R^2$, are fluorine, chlorine, bromine and iodine atom, preferably chlorine atom.

In the general formulae (IIa), (IIb), (IIc) and (IId), it is also preferable that $R^1$ or $R^2$ represents a hydrogen atom.

In the general formula (IIa), it is also preferable that $(R^1)_n$ represents hydroxy group(s).

In the general formula (IIa), preferable Z is a sulfur atom.

In the general formula (IIa), preferable $(R^1)_n$ represents that:

(i) n is an integer of 1 and $R^1$ is a hydrogen atom, a straight- or branched-chain alkyl group containing from 1 to 6 carbon atoms or a halogen atom, or n is an integer of 3 and $R^1$, which may be the same or different, is a straight- or branched-chain alkyl group containing from 1 to 6 carbon atoms;

(ii) n is an integer of 1 and $R^1$ is a hydroxy group; or

(iii) n is an integer of 2 and two $R^1$s are a hydroxy group and an amino group, such groups being at o-position or p-position to each other.

In the general formula (IIa), when $(R^1)_n$ represents the case (i) or (ii), $(R^1)_n$ may be attached to the free position of the benzene ring, and when $(R^1)_n$ represents the case (iii), $(R^1)_n$ may be attached wherever $(R^1)_n$ may be at o-position or p-position to each other. Preferably $(R^1)_n$ is attached:

(a) to the 4th position of the benzene ring when $(R^1)_n$ represents the case (i) and n is an integer of 1, and to the 2nd, 4th and 6th positions of the benzene ring when $(R^1)_n$ represents the case (i) and n is an integer of 3,

(b) to the 4th position of the benzene ring when $(R^1)_n$ represents the case (ii) and n is an integer of 1, and

(c) to the 3rd and 4th positions of the benzene ring when $(R^1)_n$ represents the case (iii).

More preferred examples of $(R^1)_n$ are a hydrogen atom, 4-methyl group, 4-chloro group, 4-hydroxy gorup, 3-amino-4-hydroxy

group, 2,4,6-trimethyl group and 4-tert-butyl group.

In the general formulae (IIb), (IIc) and (IId), preferable $(R^2)_m$ represents that m is an integer of 1 and $R^2$ is a hydrogen atom, a straight- or branched-chain alkyl group containing from 1 to 6 carbon atoms or a halogen atom.

In the general formulae (IIb), (IIc) and (IId), $(R^2)_m$ may be attached to the free position of the benzene ring or of the benzothiazole ring, and preferably attached to the 4th position of the benzene ring when m is an integer of 1 in the general formulae (IIb) and (IIc), and preferably attached to the 5th or 6th position of the benzothiazole ring when m is an integer of 1 in the general formula (IId).

In the general formulae (IIb), (IIc) and (IId), more preferred example of $(R^2)_m$ is a hydrogen atom.

Many analogous compounds to compounds of the present invention, i.e. compounds substituted by various benzene derivatives at the 4th, 5th or 6th position of 2-aminophenol, have been known well. For example, 2-amino-4-benzenesulfonylphenol [Chem. Abst., 50, 14236g (1956)], 2-amino-4-(2-benzimidazolyl)phenol [Chem. Abst., 46, 2811h (1952)], N-phenyl-3-amino-4-hydroxybenzamide [Chem. Abst., 48, 14223i (1954)], 2-amino-6-(2-benzothiazolyl)phenol [Chem. Abst., 87, 137320v (1977)] and 2-amino-5-phenylthiophenol [Chem. Abst., 76, 25208f (1972) and ibid, 78, 43391f (1973)] have been already known.

However, the compounds of the present invention are entirely novel, and further there is no literatures describing that the above compounds have the same inhibitory activity on 5-lipoxygenase or on cyclooxygenase as the compounds of the present invention possess.

The compounds of the general formula (I) may be classified, according to the difference of R, into the compounds of the general formulae (Ia), (Ib), (Ic) and (Id) below:

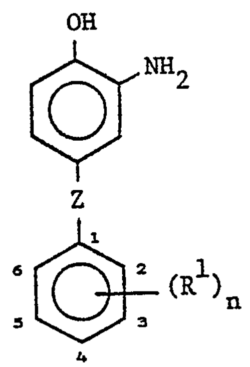

(Ia),

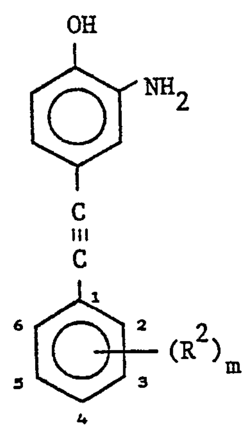

(Ib),

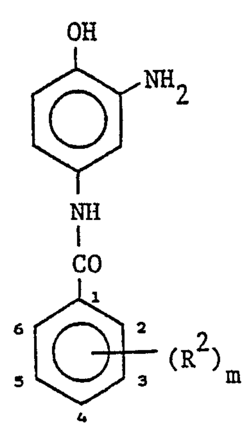

(Ic)

and

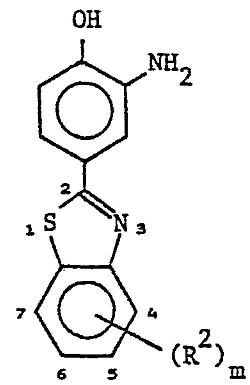

(Id)

(wherein the various symbols are as hereinbefore defined).

According to the present invention, compounds of the general formulae (Ia), (Ib), (Ic) and (Id) may be prepared by the selective reduction to amino group(s) of the nitro group (and $R^3$, when one of $R^3$ represents a nitro group) of the corresponding compounds of the following general formulae (IIIa), (IIIb), (IIIc) and (IIId), respectively, i.e. compounds of the general formulae:

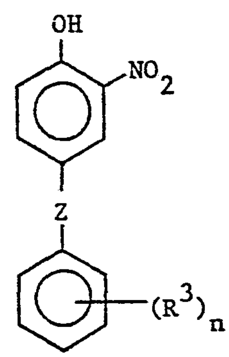

(IIIa),

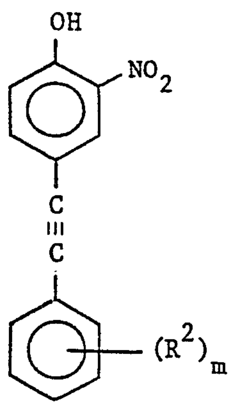

(IIIb),

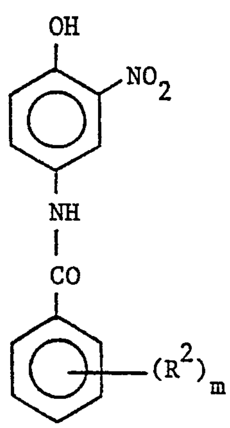

(IIIc)

and

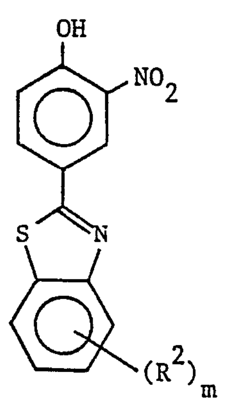

(IIId)

(wherein $(R^3)_n$ represents that:

(i) n is an integer of 1, 2 or 3 and $R^3$, which may be the same or different, is a hydrogen atom, a straight- or branched-chain alkyl group containing from 1 to 6 carbon atoms or a halogen atom;

(ii) n is an integer of 1, 2 or 3 and $R^3$ is a hydroxy group; or

(iii) n is an integer of 2 and two $R^3$s are a hydroxy group and a nitro group, a hydroxy group and a straight- or branched-chain alkyl group containing from 1 to 6 carbon atoms, or a hydroxy group and a halogen atom, such groups being at o-position or p-position to each other,

and the other symbols are as hereinbefore defined).

The reduction can be carried out, for example, by using hydrogen gas in the presence of a catalyst such as nickel, platinum or palladium-carbon, by using iron, zinc, tin or stannous chloride in addition to hydrochloric acid, by using titanium trichloride in the presence of benzene or a mixture of benzene and tetrahydrofuran, by using sodium sulfide or ammonium sulfide in water or an aqueous alcohol, or by using sodium hydrosulfite in ammonia water etc.

Compounds of the general formula (IIIa) may be prepared by the series of reactions depicted schematically below in Scheme B.

SCHEME B

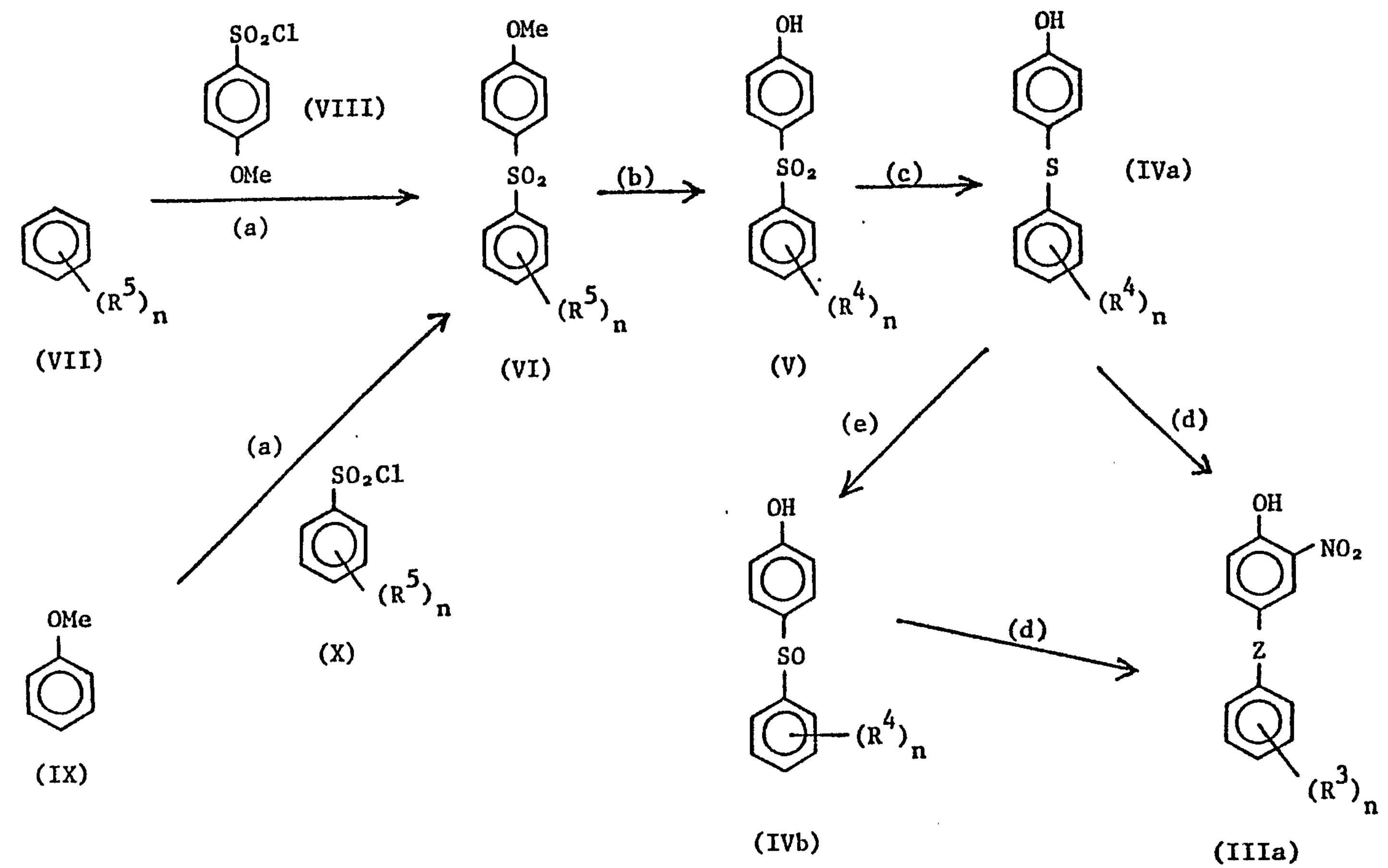


(VIII) (VII) (VI) (V) (IVa) (IX) (X) (IVb) (IIIa)

In Scheme B, $(R^4)_n$ represents that:

(i) n is an integer of 1, 2 or 3 and $R^4$, which may be the same or different, is a hydrogen atom, a straight- or branched-chain alkyl group containing from 1 to 6 carbon atoms or a halogen atom ;

(ii) n is an integer of 1, 2 or 3 and $R^4$ is a hydroxy group; or

(iii) n is an integer of 2 and two $R^4$s are a hydroxy group and a straight- or branched-chain alkyl group containing from 1 to 6 carbon atoms, or a hydroxy group and a halogen atom, such groups being at o-position or p-position to each other,

and $(R^5)_n$ represents that:

(i) n is an integer of 1, 2 or 3 and $R^5$, which may be the same or different, is a hydrogen atom a straight- or branched-chain alkyl group containing from 1 to 6 carbon atoms or a halogen atom;

(ii) n is an integer of 1, 2 or 3 and $R^5$ is a methoxy group; or

(iii) n is an integer of 2 and two $R^5$s are a methoxy group and a straight- or branched-chain alkyl group containing from 1 to 6 carbon atoms, or a methoxy group and a halogen atom, such groups being at o-position or p-position to each other,

and the other symbols are as hereinbefore defined.

Explaining each step in Scheme B, step (a) is Friedel-Crafts type sulfonation of compounds of the general formula (VII) with a compound of the formula (VIII), or of a compound of the formula (IX)

with compounds of the general formula (X). Step (a) may be carried out using a sulfonating reagent such as sulfonyl halide or acid anhydride, in the presence of a catalyst, e.g. aluminium chloride or ferric chloride in an inert organic solvent, e.g. carbon disulfide, tetrahydrofuran, methylene chloride or 1,2-dichloroethane, at a temperature of from 0°C to 50°C generally (preferably at room temperature).

Step (b) is the hydrolysis of the methoxy group (and $R^5$(s), when at least one $R^5$ represents a methoxy group) to hydroxy group(s), and may be carried out by using a hydrohalogenated acid such as hydrobromic acid and/or hydroiodic acid. Compounds of the general formula (V) can be prepared directly by effecting step (a) without effecting step (b) by varying reaction conditions or starting materials of step (a).

Step (c) is the reduction of a sulfonyl group to a thio group, and may be carried out by using a reductant such as diisobutylaluminium hydride (DIBAL).

Step (d) is the nitration, and may be carried out by using a conventional nitrating reagent, for example, a mixture of nitric acid and sulfuric acid, a mixture of nitric acid and sulfuric acid in acetic acid, or nitric acid in acetic acid.

Step (e) is the oxidation of a thio group to a sulfinyl group, and may be carried out by using m-chloroperbenzoic acid (MCPBA).

The compounds of the general formulae (VII) and (X), and the compounds of the formulae (VIII) and (IX) are well known *per se* or may be prepared by methods known *per se*. For example, 1,3,5-trimethylbenzene and tert-butylbenzene [compounds of the general formula (VII)], anisole [compound of the general formula (VII) or compound of the formula (IX)], 4-methoxybenzenesulfonyl chloride [compound of the formula (VIII) and compound of the general formula (X)] and benzenesulfonyl chloride, 4-chlorobenzenesulfonyl chloride and p-toluenesulfonyl chloride [compounds of the general formula (X)] are compounds on the market.

Compounds of the general formula (IIIb) may be prepared by the nitration of compounds of the general formula:

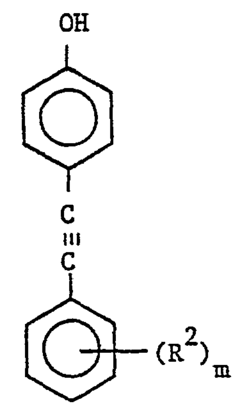

(XI)

(wherein the various symbols are as hereinbefore defined). The nitration may be carried out by methods hereinbefore described for step(d) in Scheme B.

Compounds of the general formula (XI) may be prepared by the reaction of a compound of the formula:

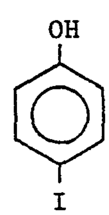

(XII)

with compounds of the general formula:

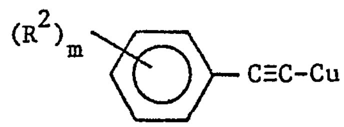

(XIII)

(wherein the various symbols are as hereinbefore defined).

Compounds of the general formula (XIII) may be prepared from compounds of the general formula:

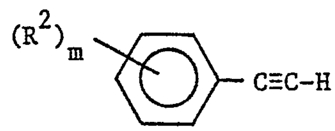

(XIV)

(wherein the various symbols are as hereinbefore defined).

Methods for the conversion of compounds of the general formula (XIV) into compounds of the general formula (XIII) and methods for the reaction of a compound of the formula (XII) with compounds of the general formula (XIII) are described in the Journal of the Organic Chemistry (J. Org. Chem.), 28, 3313 (1963) in detail.

The compound of the formula (XII) and the compounds of the general formula (XIV) are well known *per se* or may be prepared by methods known *per se*. For Example, p-iodophenol of the compound of the formula (XII), and phenylacetylene, of compounds of the general formula (XIV) are compounds on the market.

Compounds of the general formula (IIIc) may be prepared by the hydrolysis under alkaline conditions of compounds of the general formula:

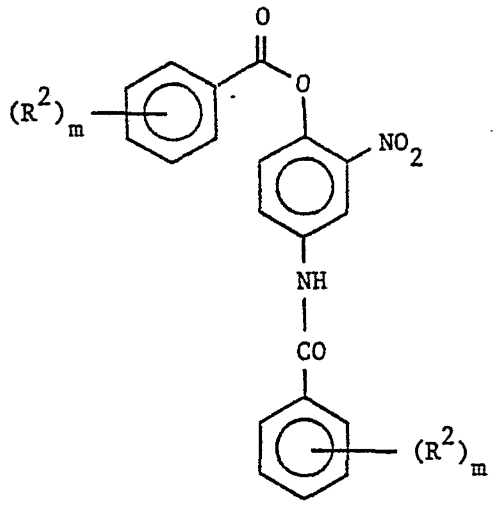

(XV)

(wherein the various symbols are as hereinbefore defined).

The hydrolysis under alkaline conditions may be effected with an aqueous solution of an alkali metal, e.g. sodium or potassium, hydroxide or carbonate in the presence of a water miscible solvent, e.g. tetrahydrofuran or a lower alkanol such as methanol or ethanol.

Compounds of the general formula (XV) may be prepared by the reaction of a compound of the formula:

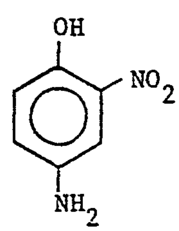

(XVI)

with compounds of the general formula:

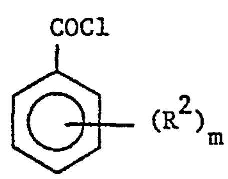

(XVII)

(wherein the various symbols are as hereinbefore defined).

The reaction may, generally, be carried out in an inert organic solvent such as methylene chloride in the presence of a base such as triethylamine at a temperature of from -70°C to 0°C.

The compound of the formula (XVI) and the compounds of the general formula (XVII) are well known per se or may be prepared by methods known per se. For example, 4-amino-2-nitrophenol of the compound of the formula (XVI), and p-chlorobenzoyl chloride and benzoyl chloride, of the compounds of the general formula (XVII) are compounds on the market.

Compounds of the general formula (IIId) may be prepared by the ring closure by reacting a compound of the formula

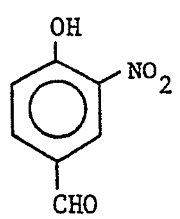

(XVIII)

with compounds of the general formula:

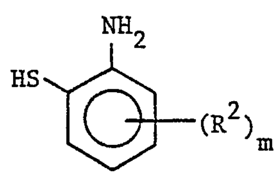

(XIX)

(wherein the various symbols are as hereinbefore defined).

The ring closure reaction are described in the Journal of the American Chemical Society (J. Am. Chem. Soc.), 53, 2654 (1931), Chemical Abstracts, 52, 13094f (1958) and Antibiotics and Chemiotherapy, 8, 33 (1958) in detail.

The compound of the formula (XVIII) and the compounds of the general formula (XIX) are well known *per se* or may be prepared by methods known *per se*. For example, 4-hydroxy-3-nitrobenzaldehyde of the compound of the formula (XVIII), and 2-aminobenzenethiol, of the compounds of the general formula (XIX) are compounds on the market.

Acid addition salts of 2-aminophenol derivatives of the general formula (I) may be prepared from the compounds of the general formula (I) by methods known *per se*, for example, by reacting stoichiometric quantities of a compound of the general formula (I) and an appropriate acid, e.g. an inorganic acid such as hydrochloric acid, hydrobromic acid, hydroiodic acid, sulfuric acid, phosphoric acid or nitric acid, or an organic acid such as acetic acid, lactic acid, tartric acid, benzoic acid, citric acid, methanesulphonic acid, ethanesulphonic acid, benzenesulphonic acid, toluenesulphonic acid or isethionic acid, in a suitable solvent.

Preferably, acid addition salts are non-toxic salts. By the term 'non-toxic' as used in this specification, is meant salts the anions of which are relatively innocuous to animal organism when used in therapeutic doses so that the benefitical pharmacological properties of the compounds of general formula (I) are not vitiated by side effects ascribable to those anions. Preferably the salts are water-soluble. Suitable acid addition salts are, for example, inorganic acid salts such as hydrochloride, hydrobromide, hydroiodide, sulfate, phosphorate, nitrate, or organic acid salts such as acetate, lactate, tartrate, benzoate, citrate, methanesulphonate, ethanesulphonate, benzenesulphonate, toluenesulphonate or isethionate.

2-Aminophenol derivatives of the general formula (I) and their pharmaceutically-acceptable acid addition salts possess an inhibitory effect on 5-lipoxygenase specifically, or 5-lipoxygenase and cyclooxygenase at the same time, and therefore, are effective when it is desired to control the biosynthesis of leukotrienes and various PGs in mammals including human, especially human.

For example, in standard assay system using polymorphonuclear leukocytes from guinea pig (details of experiments were described hereafter), (a) the concentrations of 2-amino-4-(4-methylphenylthio)phenol hydrochloride for 50% inhibitions on 5-lipoxygenase and on cyclooxygenase were 0.2 μM and 0.3 μM, respectively, (b) the concentrations of 2-amino-4-phenylthiophenol hydrochloride for 50% inhibitions on 5-lipoxygenase and on cyclooxygenase were 0.1 μM and 0.3 μM, respectively and (c) the concentrations of 2-amino-4-(4-chlorophenylthio)phenol hydrochloride for 50% inhibitions on 5-lipoxygenase and on cyclooxygenase were 0.5 μM and 0.3 μM, respectively. Therefore, it is comfirmed that these compounds (a), (b) and (c) inhibit both 5-lipoxygenase and cyclooxygenase at the same time at very low concentrations.

The experiments for screening using polymorphonuclear leukocytes were carried out as follows: The reaction mixture (0.1 ml) containing 5 μmole of potassium phosphate at pH 7.4, 0.1 μmole of calcium chloride, polymorphonuclear leukocytes prepared from guinea pigs ($4 \times 10^7$ cells) and test compound, was preincubated for 5 minutes at 37°C. The reaction was started by the addition of [$^{14}C$] arachidonic acid ($2.2 \times 10^5$ dpm, 1.8 nmole), followed by the incubation at 37°C for 5 minutes. The reaction was terminated by the addition of 0.3 ml of a mixture of ether/

methanol/1M citric acid (30/4/1). The organic phase (20 µl) was applied to silica gel plate. Thin layer chromatography was performed to separate each product with solvent system of ether/petroleum ether/acetic acid (85/15/0.1).

Activity of 5-lipoxygenase and cyclooxygenase were calculated from the measurements of the production of 5-HETE, and 12L-hydroxy-5,8,10-heptatrienoic acid (i.e. HHT) or thromboxane $B_2$ (i.e. $TXB_2$), respectively [it has been known that HHT and $TXB_2$ are the last products of the system concerning cyclooxygenase in the arachidonate cascade]. The concentrations for 50% inhibitions on 5-lipoxygenase and on cyclooxygenase were evaluated by using the concentrations of compounds of the present invention required 50% inhibitions on the productions of 5-HETE, and HHT or $TXB_2$, respectively.

Further, in another assay system evaluating only the inhibitory activity on 5-lipoxygenase using polymorphonuclear leukocytes from guinea pig (details of experiments were described hereafter), the concentrations of 2-amino-4-phenylthiophenol hydrochloride, 2-amino-4-(4-methylphenylthio)phenol hydrochloride, 2-amino-4-(4-chlorophenylthio)phenol hydrochloride, 2-amino-4-(4-hydroxyphenylthio)phenol, 2-amino-4-(3-amino-4-hydroxyphenylthio)phenol, 2-amino-4-(2,4,6-trimethylphenylthio)phenol hydrochloride, 2-amino-4-(4-tert-butylphenylthio)phenol hydrochloride, 2-amino-4-benzenesulfinylphenol, N-(3-amino-4-hydroxyphenyl)benzamide hydrochloride, 2-amino-4-(2-benzothiazolyl)phenol hydrochloride and 2-amino-4-phenylethynylphenol, required to produce 50% inhibitions on 5-lipoxygenase, were 0.31, 0.20, 0.30, 0.46, 1.5, 0.48, 0.32, 11, 18, 0.23 and 0.69 µM, respectively.

Therefore, it is confirmed that all these compounds inhibit 5-lipoxygenase at very low concentrations.

The experiments for evaluating only the inhibitory activity on 5-lipoxygenase were carried out as follows: Guinea pig was treated by casein and harvested polymorphonuclear leukocytes from cavity were sonicated, followed by the centrifugation at 105,000 x g. The supernate was used as an enzyme throughout this work. The supernate (100 - 500 μg of protein) was incubated for 5 minutes at 30°C with [$^{14}C$] arachidonic acid and 1 mM calcium chloride in the presence or in the absence of test compound. The reaction was terminated by the addition of ether. Ether extract was separated by thin layer chromatography using silica gel plates to observe production of 5-HETE, 5,12-diHETE and leukotriene B. In this assay system, the concentrations for 50% inhibitions on 5-lipoxygenase were evaluated by using the concentrations of compounds of the present invention required 50% inhibitions on the production of 5-HETE, 5,12-diHETE and leukotrien B.

On the other hand, all extent of the compounds of the present invention were confirmed that their acute toxicities were more than 30 mg/kg by intravenous administration. Therefore, 2-aminophenol derivatives of the present invention may be considered to be sufficiently safe and suitable for medical use. For example, no mouse was dead when 2-amino-4-phenylthiophenol hydrochloride, 2-amino-4-(4-methylphenylthio)-phenol hydrochloride, 2-amino-4-(2-benzothiazolyl)phenol hydrochloride and 2-amino-4-phenylethynylphenol were intravenously administered to tail vein in ten mice at the dose of 30 mg/kg, respectively.

To control the biosynthesis of leukotrienes and prostaglandins

are useful for the prevention and the treatment of allergic tracheal and bronchial diseases or allergic lung diseases, allergic shocks or various allergic inflammations, in addition to various inflammations induced by prostaglandins in mammals including human, especially human. For such purpose, the compounds of this invention are usually administered systemically, for example, orally, rectally or parenterally. Doses are determined depending upon age, symptoms, the desired therapeutic effects, the route of administration, the duration of the treatment and the like, and are generally and preferably about 0.1 mg to 500 mg for oral administration, and 1 μg to 10 mg for intravenous injection or 0.1 μg to 1 mg/hour for intravenous infusion, specially when required emergency treatment.

Solid compositions for oral administration include compressed tablets, pills, dispersible powders and granules. In such solid compositions, one or more of the active compounds is or are, admixed with at least one inert diluent such as calcium carbonate, potato starch, dextrin, alginic acid, lactose, mannitol, glucose and cacao butter. The compositions may also comprise, as is normal practice, additional substances other than inert diluents e.g. lubricating agents such as magnesium stearate or disintegrating agents such as cellulose calcium gluconate. The tablets or pill may, if desired, be coated and made into sugar-coated, gelatin-coated, enteric-coated or film-coated tablets and pills, or tablets or pills may be coated with two or more layers.

Liquid compositions for oral administration include pharmaceutically acceptable emulsions, solutions, suspensions, syrups and elixirs containing inert diluents commonly used in the art, such as

water or liquid paraffin. Besides inert diluents such compositions may also comprise adjuvants such as wetting and suspending agents, and sweetening, flavouring, perfuming and preservating agents.

The compositions according to this invention for oral administration, also include capsules of absorbable materials such as gelatin containing one or more of the active substances with or without the addition of diluents or excipients.

Solid compositions for intrarectal administration include suppositories formulated in manner known per se and containing one or more of the active compounds.

Preparation according to this invention for parenteral administration include sterile aqueous or non-aqueous solutions, suspensions or emulsions. Examples of non-aqueous solvents or suspending media are propylene glycol, polyethylene glycol, ethanol, vegetable oils such as olive oil, and injectable organic esters such as ethyl oleate or sorbitan esters. These compositions may also include adjuvants such as preserving, wetting, emulsifying and dispersing agents. They may be sterilized, for example, by filtration through a bacteria-retaining filter, by incorporation of sterilizing agents in the compositions or by irradiation. They may also be manufactured in the form of sterile solid compositions which can be dissolved in sterile water or some other sterile injectable medium immediately before use.

The percentage of active ingredient in the compositions of the present invention may be varied, it being necessary that it should constitute a proportion such that a suitable dosage for the desired therapeutic effect shall be obtained. Several unit dosage forms may of

course be administered at the same time. In general the preparations should normally contain at least 0.025% by weight of active substance when required for administration by injection and the preparations should normally contain at least 0.1% by weight of active substance when required for oral administration.

Preferred compounds of the present invention are, for example, as follows:

(a) as compounds of the general formula (Ia):

2-amino-4-phenylthiophenol,

2-amino-4-(4-methylphenylthio)phenol,

2-amino-4-(4-ethylphenylthio)phenol,

2-amino-4-(4-propylphenylthio)phenol,

2-amino-4-(4-butylphenylthio)phenol,

2-amino-4-(4-tert-butylphenylthio)phenol,

2-amino-4-(4-pentylphenylthio)phenol,

2-amino-4-(4-hexylphenylthio)phenol,

2-amino-4-(2,4,6-trimethylphenylthio)phenol,

2-amino-4-(4-fluorophenylthio)phenol,

2-amino-4-(4-chlorophenylthio)phenol,

2-amino-4-(4-bromophenylthio)phenol,

2-amino-4-(4-iodophenylthio)phenol,

2-amino-4-(4-hydroxyphenylthio)phenol,

2-amino-4-(3-amino-4-hydroxyphenylthio)phenol,

2-amino-4-benzenesulfinylphenol,

2-amino-4-(4-methylbenzenesulfinyl)phenol,

2-amino-4-(4-ethylbenzenesulfinyl)phenol,

2-amino-4-(4-propylbenzenesulfinyl)phenol,

2-amino-4-(4-butylbenzenesulfinyl)phenol,

2-amino-4-(4-tert-butylbenzenesulfinyl)phenol,

2-amino-4-(4-pentylbenzenesulfinyl)phenol,

2-amino-4-(4-hexylbenzenesulfinyl)phenol,

2-amino-4-(2,4,6-trimethylbenzenesulfinyl)phenol,

2-amino-4-(4-fluorobenzenesulfinyl)phenol,

2-amino-4-(4-chlorobenzenesulfinyl)phenol,

2-amino-4-(4-bromobenzenesulfinyl)phenol,

2-amino-4-(4-iodobenzenesulfinyl)phenol,

2-amino-4-(4-hydroxybenzenesulfinyl)phenol,

2-amino-4-(3-amino-4-hydroxybenzenesulfinyl)phenol,

and acid addition salts thereof;

(b) as compounds of the general formula (Ib):

2-amino-4-phenylethynylphenol

2-amino-4-(4-methylphenylethynyl)phenol,

2-amino-4-(4-ethylphenylethynyl)phenol,

2-amino-4-(4-propylphenylethynyl)phenol,

2-amino-4-(4-butylphenylethynyl)phenol,

2-amino-4-(4-tert-butylphenylethynyl)phenol,

2-amino-4-(4-pentylphenylethynyl)phenol,

2-amino-4-(4-hexylphenylethynyl)phenol,

2-amino-4-(4-fluorophenylethynyl)phenol,

2-amino-4-(4-chlorophenylethynyl)phenol,

2-amino-4-(4-bromophenylethynyl)phenol,

2-amino-4-(4-iodophenylethynyl)phenol,

and acid addition salts thereof;

(c) as compounds of the general formula (Ic):

N-(3-amino-4-hydroxyphenyl)benzamide,

N-(3-amino-4-hydroxyphenyl)-4-methylbenzamide,

N-(3-amino-4-hydroxyphenyl)-4-ethylbenzamide,

N-(3-amino-4-hydroxyphenyl)-4-propylbenzamide,

N-(3-amino-4-hydroxyphenyl)-4-butylbenzamide,

N-(3-amino-4-hydroxyphenyl)-4-tert-butylbenzamide,

N-(3-amino-4-hydroxyphenyl)-4-pentylbenzamide,

N-(3-amino-4-hydroxyphenyl)-4-hexylbenzamide,

N-(3-amino-4-hydroxyphenyl)-4-fluorobenzamide,

N-(3-amino-4-hydroxyphenyl)-4-chlorobenzamide,

N-(3-amino-4-hydroxyphenyl)-4-bromobenzamide,

N-(3-amino-4-hydroxyphenyl)-4-iodobenzamide,

and acid addition salts thereof; and

(d) as compounds of the general formula (Id):

2-amino-4-(2-benzothiazolyl)phenol,

2-amino-4-[2-(5-methylbenzothiazolyl)]phenol,

2-amino-4-[2-(6-methylbenzothiazolyl)]phenol,

2-amino-4-[2-(5-ethylbenzothiazolyl)]phenol,

2-amino-4-[2-(6-ethylbenzothiazolyl)]phenol,

2-amino-4-[2-(5-propylbenzothiazolyl)]phenol,

2-amino-4-[2-(6-propylbenzothiazolyl)]phenol,

2-amino-4-[2-(5-butylbenzothiazolyl)]phenol,

2-amino-4-[2-(6-butylbenzothiazolyl)]phenol,

2-amino-4-[2-(5-tert-butylbenzothiazolyl)]phenol,

2-amino-4-[2-(6-tert-butylbenzothiazolyl)]phenol,

2-amino-4-[2-(5-pentylbenzothiazolyl)]phenol,

2-amino-4-[2-(6-pentylbenzothiazolyl)]phenol,

2-amino-4-[2-(5-hexylbenzothiazolyl)]phenol,

2-amino-4-[2-(6-hexylbenzothiazolyl)]phenol,

2-amino-4-[2-(5-fluorobenzothiazolyl)]phenol,

2-amino-4-[2-(6-fluorobenzothiazolyl)]phenol,

2-amino-4-[2-(5-chlorobenzothiazolyl)]phenol,

2-amino-4-[2-(6-chlorobenzothiazolyl)]phenol,

2-amino-4-[2-(5-bromobenzothiazolyl)]phenol,

2-amino-4-[2-(6-bromobenzothiazolyl)]phenol,

2-amino-4-[2-(5-iodobenzothiazolyl)]phenol,

2-amino-4-[2-(6-iodobenzothiazolyl)]phenol,

and acid addition salts thereof.

The following Reference Examples and Examples illustrate, but not limit, the preparation of compounds of the present invention. In the Reference Examples and Examples, 'TLC', 'IR', 'NMR' and 'Mass' represent 'Thin layer chromatography', 'Infrared absorption spectrum', 'Nuclear magnetic resonance' and 'Mass spectrum', respectively. Where solvent ratios are specified in chromatographic separations, the ratios are by volume. The solvents in parentheses in 'TLC' show the developing solvent used. Except when specified otherwise, 'IR' is recorded by KBr tablet method and 'NMR' is recorded in deuterochloroform ($CDCl_3$) solution.

Reference Example 1

4-Benzenesulfonyl anisole

14 g of aluminium chloride was finely crushed and dispersed in 50 ml of 1,2-dichloroethane, and 14.7 g of benzenesulfonyl chloride (being on the market) was added thereto. The mixture was stirred for 15 minutes at room temperature. To the reaction mixture, 10 g of anisole in 50 ml of 1,2-dichloroethane was added dropwise over a period of 30 minutes. The mixture was stirred for 110 hours at room temperature. The reaction mixture was poured into ice-hydrochloric acid and extracted with chloroform. The extract was washed with water and a saturated aqueous solution of sodium chloride, successively, dried over anhydrous magnesium sulfate and concentrated under reduced pressure. The residue was purified by column chromatography on silica gel using benzene as an eluent to give 9.2 g of the title compound (containing para isomer in part) having the following physical characteristics:

Melting point: 125 - 135°C;

TLC (benzene:ethyl acetate=2:1): Rf=0.25;

NMR: δ=7.77 (2H, m), 7.62 (2H, d), 7.35 (3H, m), 6.83 (2H, d);

Mass: m/e=248 ($M^+$), 155, 123, 107, 95, 92, 77.

By the same procedure as described in Reference Example 1, the compounds having the following physical characteristics were obtained from anisol and the corresponding sulfonyl chloride.

(a) 4-(p-Toluenesulfonyl)anisole

Starting materials: 5 g of anisole and 9.7 g of p-toluenesulfonyl chloride (being on the market);

Yield: 8.5 g (containing para isomer in part);

NMR: δ=8.0-6.7 (8H, m), 3.70 (3H, s), 3.67 (3H, s), 2.40 (3H, s), 2.30 (3H, s).

(b) 4-(4-Chlorobenzenesulfonyl)anisole

Starting materials: 5 g of anisole and 10.7 g of 4-chlorobenzenesulfonyl chloride (being on the market);

Yield: 5 g (containing para isomer in part);

NMR: δ=8.1-6.7 (8H, m), 3.75 (3H, s), 3.70 (3H, s).

(c) 4-(4-Methoxybenzenesulfonyl)anisole

Starting materials: 5 g of anisole and 9.6 g of 4-methoxybenzenesulfonyl chloride (being on the market);

Yield: 13 g (containing para isomer in part);

NMR: δ=8.2-6.6 (8H, m), 3.6 (6H, s).

(d) (1) 4-(2,4,6-Trimethylbenzenesulfonyl)phenol and

(2) 4-(2,4,6-Trimethylbenzenesulfonyl)anisole

Starting materials: 3.2 g of 1,3,5-trimethylbenzene (being on the market) and 5 g of 4-methoxybenzenesulfonyl chloride (being on the market) [The products (1) and (2) were prepared from the same starting materials];

(1) Product (1)

Yield: 2.4 g;

IR: ν=3350, 2990, 2940, 1600, 1590, 1500, 1440, 1405, 1385, 1285, 1230, 1130, 1105 (shoulder), 1100, 1050, 840, 720, 690 $cm^{-1}$;

NMR (deuterochloroform + acetone-$d_6$ solution): δ=7.51 (2H, d), 6.82 (2H, d), 6.81 (2H, s), 2.53 (6H, s), 2.23 (3H, s);

Mass: m/e=276 ($M^+$), 258, 241, 220, 195, 171, 165, 91.

(2) Product (2)

Yield: 4 g;

Mass: m/e=290 ($M^+$), 272, 255, 234, 211, 209, 195, 165, 91.

(e) 4-(4-tert-Butylbenzenesulfonyl)anisole

Starting materials: 6.5 g of tert-butylbenzene (being on the market) and 5 g of 4-methoxybenzenesulfonyl chloride (being on the market);

Catalyst: ferric chloride instead of aluminium chloride;

Yield: 10 g;

NMR: δ=7.73 (2H, d), 7.70 (2H, d), 7.35 (2H, d), 6.82 (2H, d), 3.73 (3H, s), 1.3 (9H, s);

Mass: m/e=304 ($M^+$), 289, 275, 225, 198, 155, 123, 107, 77.

Reference Example 2

4-Benzenesulfonylphenol

To 9.2 g of 4-benzenesulfonyl anisole (prepared in Reference Example 1) was added 100 ml of a 47% aqueous solution of hydrobromic acid and the mixture was refluxed with heating for 6 hours. The reaction mixture was cooled to room temperature and extracted with diethyl ether. The extract was washed with an aqueous solution of sodium thiosulfate, water and a saturated aqueous solution of sodium chloride, successively, dried over anhydrous magnesium sulfate and concentrated under reduced pressure. The crude crystal thus obtained (10 g) was purified by column chromatography on silica gel using a mixture of benzene-ethyl acetate (10:1) as an eluent to give 4 g of the title compound having the following physical characteristics:

Melting point: 141-142°C;

TLC (benzene:ethyl acetate=2:1): Rf=0.5;

IR: ν=3400, 3070, 1600, 1590, 1500, 1440, 1310, 1290, 1240, 1155, 1110, 850, 740 $cm^{-1}$;

NMR (acetone-$d_6$ solution): δ=9.4 (1H, br-s), 7.9 (2H, m), 7.86 (2H, d), 7.6 (3H, m), 7.03 (2H, d);

Mass: m/e=234 ($M^+$), 170, 141, 126, 109, 93.

By the same procedure as described in Reference Example 2, the phenol compounds having the following physical characteristics were obtained from the corresponding anisole compounds.

(a) 4-(p-Toluenesulfonyl)phenol

Starting material: 8.5 g of 4-(p-toluenesulfonyl)anisole [prepared in Reference Example 1(a)];

Yield: 1.75 g;

TLC (benzene:ethyl acetate=10:1): Rf=0.22;

IR: ν=3380, 3070, 1600, 1585, 1500, 1440, 1305, 1290, 1230, 1145, 1110, 1075, 840, 815 $cm^{-1}$;

NMR (deuterochloroform + acetone-$d_6$ solution): δ=8.4 (1H, br-s), 7.60 (4H, d), 7.13 (2H, d), 6.83 (2H, d), 2.30 (3H, s);

Mass: m/e=248 ($M^+$), 141, 140, 139, 109.

(b) 4-(4-Chlorobenzenesulfonyl)phenol

Starting material: 5 g of 4-(4-chlorobenzenesulfonyl)anisole [prepared in Reference Example 1(b)];

Yield: 2.18 g;

TLC (chloroform): Rf=0.15;

NMR (deuterochloroform + acetone-$d_6$ solution): δ=8.81 (1H, br-s), 8.73 (2H, d), 7.67 (2H, d), 7.37 (2H, d), 6.90 (2H, d).

(c) 4-(4-Hydroxybenzenesulfonyl)phenol

Starting material: 13 g of 4-(4-methoxybenzenesulfonyl)anisole [prepared in Reference Example 1(c)];

Yield: 4.4 g;

TLC (benzene:ethyl acetate=2:1): Rf=0.2;

IR: ν=3400, 1600, 1585, 1500, 1440, 1370, 1290, 1220, 1180, 1140, 1110, 1075, 1010, 850, 840, 820, 725, 690 $cm^{-1}$;

NMR (deuterochloroform + methanol-$d_4$ + acetone-$d_6$ solution): δ=7.60 (4H, d), 6.80 (4H, d);

Mass: m/e=250 ($M^+$), 142, 141, 110, 93.

(d) 4-(4-tert-Butylbenzenesulfonyl)phenol

Starting material: 10 g of 4-(4-tert-butylbenzenesulfonyl)anisole [prepared in Reference Example 1(e)];

Yield: 7.1 g;

IR: ν=3400, 3060, 2970, 2870, 1600, 1580, 1500, 1475, 1435, 1395, 1365, 1305, 1290, 1220, 1145, 1115, 1095, 1070, 1010, 835, 760 $cm^{-1}$;

NMR: δ=7.68 (2H, d), 7.63 (2H, d), 7.35 (2H, d), 6.80 (2H, d), 1.27 (9H, s);

Mass: m/e=290 ($M^+$), 275, 261, 211, 181, 166, 157, 141, 135.

Reference Example 3

4-Phenylthiophenol

A solution of 1.7 g of 4-benzenesulfonylphenol (prepared in Reference Example 2) in 10 ml of toluene was cooled under ice and thereto was added slowly 29 ml of a 1.76N solution of diisobutylaluminium hydride (DIBAL). After the completion of the evolution of hydrogen gas, the mixture was refluxed with heating for 24 hours. The reaction mixture was cooled under ice and to the solution was added water to decompose excess amount of DIBAL. The reaction mixture was adjusted to pH 4 with 6N hydrochloric acid and extracted with ethyl acetate. The extract was washed with a saturated aqueous solution of sodium bicarbonate and a saturated aqueous solution of sodium chloride, successively, dried over anhydrous magnesium sulfate and concentrated under reduced pressure. The residue was purified by column chromatography on silica gel using a mixture of benzene-ethyl acetate (10:1) as an eluent to give 900 mg of the title compound having the following physical characteristics:

TLC (benzene:ethyl acetate=10:1): Rf=0.48.

IR (liquid film): ν=3400, 3050, 1600, 1580, 1490, 1475, 1435, 1170, 830, 740, 690 $cm^{-1}$;

NMR ($CCl_4$ solution): δ=7.28 (2H, d), 7.1 (5H, m), 6.72 (2H, d), 5.03 (1H, s);

Mass: m/e=202 ($M^+$), 183, 173, 141, 125, 115.

By the same procedure as described in Reference Example 3, the thio compounds having the following physical characteristics were obtained from the corresponding sulfonyl compounds.

(a) 4-(4-Methylphenylthio)phenol

Starting material: 1.75 g of 4-(p-toluenesulfonyl)phenol [prepared in Reference Example 2(a)];

Yield: 1.5 g;

TLC (chloroform:ethyl acetate=5:1): Rf=0.8;

NMR: δ=7.20 (2H, d), 7.01 (4H, s), 6.70 (2H, d), 2.27 (3H, s);

Mass: m/e=216 ($M^+$), 201, 183, 171, 155, 91.

(b) 4-(4-Chlorophenylthio)phenol

Starting material: 2.18 g of 4-(4-chlorobenzenesulfonyl)phenol [prepared in Reference Example 2(b)];

Yield: 1.28 g;

TLC (chloroform): Rf=0.2;

IR: ν=3350, 1595 (shoulder), 1585, 1490, 1480, 1435, 1390, 1215, 1170, 1100, 1010, 820 $cm^{-1}$;

NMR: δ=7.25 (2H, d), 7.05 (4H, m), 6.72 (2H, d);

Mass: m/e=204, 201, 200, 183, 175, 171, 125.

(c) 4-(4-Hydroxyphenylthio)phenol

Starting material: 2 g of 4-(4-hydroxybenzenesulfonyl)phenol [prepared in Reference Example 2(c)];

Yield: 1 g;

IR: ν=3290, 1600 (shoulder), 1585, 1490, 1440, 1380, 1235, 1170, 1100, 1010, 810 $cm^{-1}$;

NMR (deuterochloroform + acetone-$d_6$ solution): δ=7.05 (4H, d), 6.72 (4H, d);

Mass: m/e=218 ($M^+$), 126.

(d) 4-(2,4,6-Trimethylphenylthio)phenol

Starting material: 2 g of 4-(2,4,6-trimethylbenzenesulfonyl)phenol [prepared in Reference Example 1(d)-(1)];

Yield: 1.94 g;

IR (liquid film): ν=3320, 3030, 2920, 1600, 1585, 1490, 1460, 1435, 1380, 1240, 1170, 1080, 1060, 1030, 1010, 850, 825 $cm^{-1}$;

NMR: δ=7.2-6.5 (6H, m), 2.3 (6H, s), 2.2 (3H, s);

Mass: m/e=244 ($M^+$), 152, 150, 119, 91.

(e) 4-(4-tert-Butylphenylthio)phenol

Starting material: 2.2 g of 4-(4-tert-butylbenzenesulfonyl)phenol [prepared in Reference Example 2(d)];

Yield: 1.6 g;

IR (liquid film): ν=3400, 2960, 1595, 1580, 1490, 1425, 1395, 1360, 1270, 1230, 1165, 1120, 1095, 1010, 965, 820 $cm^{-1}$;

NMR: δ=7.4-7.0 (6H, m), 6.67 (2H, d), 1.33 (9H, s);

Mass: m/e=258 ($M^+$), 243, 236, 227, 221, 166, 151, 123, 94.

Reference Example 4

2-Nitro-4-phenylthiophenol

To a solution of 1.7 g of 4-phenylthiophenol (prepared in

Reference Example 3) in 5 ml of acetic acid, was added 0.79 g of concentrated nitric acid over a period of 15 minutes cooling under ice. After the removal of ice bath, the mixture was stirred for 30 minutes at room temperature and extracted with chloroform. The extract was washed with a saturated aqueous solution of sodium chloride, dried over anhydrous magnesium sulfate and concentrated under reduced pressure. The residue was purified by column chromatography on silica gel using a mixture of benzene-hexane (1:1) as an eluent to give 800 mg of the title compound having the following physical characteristics:

TLC (benzene:hexane=1:1): Rf=0.37;

IR (liquid film): ν=3250, 3080, 1615, 1575, 1530, 1475, 1440, 1415, 1315, 1245, 1185, 1145, 1100, 1070, 1025, 895, 830 $cm^{-1}$;

NMR: δ=10.38 (1H, s), 7.99 (1H, d), 7.30 (1H, dd), 7.2 (5H, m), 6.97 (1H, d);

Mass: m/e=247 ($M^+$), 201, 183, 171, 139, 128, 115, 109, 95.

By the same procedure as described in Reference Example 4, the nitro compounds having the following physical characteristics were obtained from the corresponding thio compounds.

(a) 4-(4-Methylphenylthio)-2-nitrophenol

Starting material: 1.5 g of 4-(4-methylphenylthio)phenol [prepared in Reference Example 3(a)];

Yield: 740 mg (red crystal);

TLC (benzene): Rf=0.6;

IR: ν=3450, 3180, 3090, 2920, 1625, 1575, 1515, 1475, 1410, 1315, 1230, 1180, 1145, 895, 835, 815 $cm^{-1}$;

NMR: δ=10.54 (1H, s), 8.05 (1H, d), 7.48 (1H, dd), 7.2 (4H, m), 7.17

(1H, d), 2.35 (3H, s);

Mass: m/e=261 ($M^+$), 215, 200, 185, 171, 123, 91.

(b) 4-(4-Chlorophenylthio)-2-nitrophenol

Starting material: 1.28 g of 4-(4-chlorophenylthio)phenol [prepared in Reference Example 3(b)];

Yield: 800 mg;

TLC (benzene): Rf=0.7;

IR: ν=3420, 3220, 3070, 1615, 1570, 1525, 1470, 1410, 1305, 1235, 1180, 1140, 1090, 1010, 830 $cm^{-1}$;

NMR: δ=10.43 (1H, s), 8.03 (1H, d), 7.42 (1H, dd), 7.15 (4H, s), 7.05 (1H, d);

Mass: m/e=281, 235, 200, 171.

(c) (1) 4-(4-Hydroxyphenylthio)-2-nitrophenol and

(2) 4-(4-Hydroxy-3-nitrophenylthio)-2-nitrophenol

Starting material: 1.0 g of 4-(4-hydroxyphenylthio)phenol [prepared in Reference Example 3(c)];

(1) Product (1)

Yield: 210 mg;

TLC (benzene:ethyl acetate=10:1): Rf=0.33;

IR: ν=3460, 1615, 1595, 1580, 1525, 1490, 1470, 1425, 1410, 1310, 1270, 1235, 1210, 1145, 1095, 1010, 950, 895, 850, 830, 760, 720 $cm^{-1}$;

NMR (deuterochloroform + methanol-$d_4$ solution): δ=7.71 (1H, d), 7.4-6.8 (6H, m);

Mass: m/e=263 ($M^+$), 217, 199, 187, 171, 155, 125.

(2) Product (2)

Yield: 120 mg;

TLC (benzene:ethyl acetate=10:1): Rf=0.52;

IR: ν=3450, 3280, 1610, 1570, 1525, 1470, 1410, 1355, 1320, 1255, 1180, 1100, 900, 835, 820, 770 $cm^{-1}$;

NMR (deuterochloroform + methanol-$d_4$ solution): δ=8.0 (2H, d), 7.50 (2H, dd), 7.07 (2H, d);

Mass: m/e=308 ($M^+$), 262, 233, 214, 196, 170, 158, 123, 115, 95.

(d) 2-Nitro-4-(2,4,6-trimethylphenylthio)phenol

Starting material: 1.9 g of 4-(2,4,6-trimethylphenylthio)phenol [prepared in Reference Example 3(d)];

Yield: 600 mg;

IR: ν=3280, 3070, 2970, 2920, 1610, 1565, 1525, 1470, 1410, 1360, 1315, 1250, 1165, 1140, 1100, 845, 760, 710 $cm^{-1}$;

NMR: δ=10.17 (1H, s), 7.50 (1H, d), 7.07 (1H, dd), 6.90 (2H, s), 6.85 (1H, d), 2.33 (6H, s), 2.28 (3H, s);

Mass: m/e=289 ($M^+$), 228, 213, 199, 150, 119, 105, 91.

(e) 4-(4-tert-Butylphenylthio)-2-nitrophenol

Starting material: 1.6 g of 4-(4-tert-butylphenylthio)phenol [prepared in Reference Example 3(e)];

Yield: 707 mg;

IR (liquid film): ν=3240, 3070, 2960, 1615, 1570, 1525, 1470, 1410, 1360, 1315, 1240, 1180, 1140, 1115, 1010, 895, 820, 760 $cm^{-1}$;

NMR: δ=10.37 (1H, s), 7.98 (1H, d), 7.42 (1H, dd), 7.20 (4H, s), 6.97 (1H, d), 1.30 (9H, s);

Mass: m/e=303 ($M^+$), 288, 274, 260, 242, 224, 213, 199, 184, 170, 165, 144, 130, 117, 91.

Reference Example 5

4-Benzenesulfinyl-2-nitrophenol

A solution of 460 mg of 2-nitro-4-phenylthiophenol (prepared in Reference Example 4) in 10 ml of methylene chloride was cooled to -72°C. To the solution was added dropwise over a period of 20 minutes a solution of 402 mg of m-chloroperbenzoic acid (MCPBA, 80%) in 20 ml of methylene chloride cooled in -50°C. The mixture was reacted for 30 minutes at -72°C and a saturated aqueous solution of sodium bicarbonate was added thereto. After the removal of ice bath, the reaction mixture was poured into a separatory funnel, and the organic layer was dried over anhydrous magnesium sulfate and concentrated under reduced pressure. The residue was purified by column chromatography on silica gel using a mixture of chloroform-ethyl acetate (5:1) as an eluent to give 215 mg of the title compound having the following physical characteristics:

IR (liquid film): ν=3260, 3070, 1615, 1580, 1530, 1475, 1440, 1420, 1325, 1255, 1190, 1160, 1100, 1085, 1045, 900, 835 $cm^{-1}$;

NMR: δ=10.17 (1H, br-s), 8.33 (1H, d), 7.61 (1H, dd), 7.4 (5H, m), 7.12 (1H, d);

Mass: m/e=263 ($M^+$), 247, 215, 186, 170, 154, 125, 110, 94.

Reference Example 6

N-(4-Benzoyloxy-3-nitrophenyl)benzamide

To a solution of 1.26 g of 4-amino-2-nitrophenol (being on the market) in 43 ml of methylene chloride was added 2.1 g of

triethylamine and the mixture was cooled to -50°C. To the mixture was added dropwise 3.0 g of benzoyl chloride and the mixture was stirred for 30 minutes at -50°C, and further for 15 hours at room temperature. The reaction mixture was extracted with chloroform and the extract was washed with an aqueous solution of sodium bicarbonate and a saturated aqueous solution of sodium carbonate, successively, dried over anhydrous magnesium sulfate and concentrated under reduced pressure. The residue was purified by column chromatography on silica gel using methylene chloride as an eluent to give 500 mg of the title compound having the following physical characteristics:

NMR (deuterochloroform + acetone-$d_6$ solution): δ=7.9 (5H, m), 7.35 (8H, m);

Mass: m/e=362 ($M^+$), 258, 228, 105, 77.

Reference Example 7

N-(4-Hydroxy-3-nitrophenyl)benzamide

To a solution of 500 mg of N-(4-benzoyloxy-3-nitrophenyl) benzamide (prepared in Reference Example 6) in 50 ml of ethanol was added 30 ml of a 1N aqueous solution of sodium hydroxide at room temperature and the mixture was stirred for 2 hours. The reaction mixture was neutralized with 1.8 ml of acetic acid and concentrated under reduced pressure. To the residue thus obtained were added chloroform and water and the mixture was extracted. The extract (the organic layer) was washed with a saturated aqueous solution of sodium chloride, dried over anhydrous magnesium sulfate and concentrated under reduced pressure. The residue was purified by column chromatography on silica gel using chloroform as an eluent and

recrystallized from ethanol to give 96 mg of the title compound having the following physical characteristics:

TLC (methylene chloride:methanol=10:1): Rf=0.75;

IR: ν=3450, 2920, 1650, 1630 (shoulder), 1520, 1480, 1410, 1340, 1310, 1235, 1070, 870, 760, 710 $cm^{-1}$;

NMR (deuterochloroform + acetone-$d_6$ solution): δ=10.30 (1H, s), 9.07 (1H, br-s), 8.45 (1H, d), 7.95 (1H, dd), 7.83 (2H, m), 7.40 (3H, m), 7.10 (1H, d);

Mass: m/e=258 ($M^+$), 153, 105, 77.

Reference Example 8

4-(2-Benzothiazolyl)-2-nitrophenol

A solution of 653 mg of 4-hydroxy-3-nitrobenzaldehyde (being on the market) and 489 mg of 2-aminobenzenethiol (being on the market) in 5 ml of pyridine was refluxed with heating for 3 hours. After cooled with water, the reaction mixture was adjusted to pH 5 by adding 20 ml of a 20% aqueous solution of hydrochloric acid and 0.5 ml of concentrated hydrochloric acid, successively. Further stirring led to the precipitation of gummy substance. The supernatant solution was removed by decantation and to the residue was added 20 ml of n-butyl alcohol and the solution was refluxed with heating for 30 minutes with blowing air. After cooling, the presipitating pale yellow solid was collected and dried to give 396 mg of the title compound having the following physical characteristics:

TLC (methylene chloride): Rf=0.68;

IR: ν=1623, 1540, 1475, 1322, 1275, 1182, 762 $cm^{-1}$;

NMR (dimethylsulfoxide-$d_6$ + deuterochloroform solution): δ=8.61 (1H, d), 8.23 (1H, dd), 8.04 (2H, m), 7.58-7.40 (2H, m), 7.33 (1H, d);
Mass: m/e=272 ($M^+$), 226, 197.

Reference Example 9

Cupper phenylacetylide

Under an atmosphere of nitrogen, to a solution of 5 g of cupric sulfate pentahydrate in 20 ml of a concentrated ammonia water was added 80 ml of water and 2.78 g of hydroxyamine hydrochloride. To the obtained solution, its temperature kept constantly at 20°C, a solution of 2.2 ml of phenylacetylene (being on the market) in 100 ml of ethanol was added dropwise slowly to precipitate instantly yellow crystal. Further, the solution was stirred for 30 minutes under cooling with ice-water. The precipitated crystal thus obtained was filtered off, washed with water, ethanol and diethyl ether, successively, and dried by vacuum pump to give 2.73 g of the title compound.

Reference Example 10

4-Phenylethynylphenol

Under an atmosphere of nitrogen, 25 ml of pyridine was refluxed with heating for 18 hours in order to degas. To pyridine thus obtained was added 510 mg of cupper phenylacetylide (prepared in Reference Example 9) and 600 mg of 4-iodophenol (being on the market). The mixture was refluxed with heating for 7 hours. The reaction mixture was poured into ice-water and extracted with diethyl ether. The extract was washed with water, dried over anhydrous magnesium sulfate and

concentrated under reduced pressure. The residue was purified by column chromatography on silica gel using a mixture of cyclohexane-ethyl acetate (10:1) as an eluent to give 139 mg of the title compound having the following physical characteristics:

TLC (cyclohexane:ethyl acetate=4:1): Rf=0.22;

IR (liquid film): ν=3350, 2200, 1700, 1600, 1585, 1500, 1260, 1215 $cm^{-1}$;

NMR: δ=6.5-7.7 (10H, m);

Mass: m/e=194 ($M^+$).

Reference Example 11

2-Nitro-4-phenylethynylphenol

By the same procedure as described in Reference Example 4, 48 mg of the title compound having the following physical characteristics was obtained using 114 mg of 4-phenylethynylphenol (prepared in Reference Example 10) instead of 4-phenylthiophenol.

TLC (cyclohexane:ethyl acetate=4:1): Rf= 0.45;

IR: ν=1620, 1530, 1490, 1320, 1240, 1180, 760, 680 $cm^{-1}$;

NMR: δ=10.68 (1H, s), 8.31 (1H, d), 7.8-7.7 (1H, m), 7.7-7.5 (2H, m), 7.5-7.3 (3H, m), 7.18 (1H, d);

Mass: m/e=239 ($M^+$).

Example 1

2-Amino-4-phenylthiophenol hydrochloride

A mixture of 530 mg of 2-nitro-4-phenylthiophenol (prepared in Reference Example 4) and 9.8 ml of a 20% aqueous solution of titanium trichloride was stirred for 3 days at room temperature.

After the completion of the reaction, a mixture of an ammonia water and chloroform was added to the reaction mixture and the mixture was extracted. The extract (the organic layer) was washed with a saturated aqueous solution of sodium chloride, dried over anhydrous magnesium sulfate and concentrated under reduced pressure. The residue was purified by column chromatography on silica gel using a mixture of methylene chloride-methanol (10:1) to give the title compound as free amine. The compound was dissolved in methanol and to the solution was added a mixture of dry hydrogen chloride and ethanol to give the title compound as salt. The crude crystal thus obtained was treated with activated carbon and concentrated under reduced pressure to give 170 mg of the title compound having the following physical characteristics:

Melting point: more than 150°C (decomposed);

TLC (methylene chloride:methanol:ammonia water=60:6:1): Rf=0.19;

IR: ν=3450, 3170, 2930, 2600, 1625, 1600, 1575, 1500, 1480, 1440, 1425, 1340, 1295, 1235, 1180, 1125, 1100, 1085, 1025, 830, 740, 690 $cm^{-1}$;

NMR (methanol-$d_4$ solution): δ=7.38 (2H, m), 7.24 (5H, s), 7.05 (1H, d);

Mass: m/e=217 ($M^+$), 199, 171, 156.

Example 2

2-Amino-4-(4-methylphenylthio)phenol hydrochloride

To a dispersion liquid of 730 mg of powdered 4-(4-methylphenylthio)-2-nitrophenol [prepared in Reference Example 4(a)] in 12 ml of water, 10 ml of a concentrated ammonia water and 2 ml of tetrahydrofuran were added to obtain a homogeneous solution. To the solution was added a solution of 5 g of sodium hydrosulfide in 20 ml

of water. After the reaction mixture changed from red to yellow, it was extracted with diethyl ether. The extract was washed with a saturated aqueous solution of sodium chloride, dried over anhydrous magnesium sulfate and concentrated under reduced pressure. The crude product thus obtained was dissolved in ethanol and thereto was added 0.2 ml of a concentrated hydrochloric acid and the mixture was concentrated under reduced pressure and further dried by vacuum pump. The crude crystal thus obtained was dissolved in ethanol and reprecipitated by adding diethyl ether to give 700 mg of the title compound as crystal having the following physical characteristics:

Melting point: more than 170°C (decomposed);

TLC (methylene chloride:methanol=10:1): Rf=0.58;

IR: $\nu$=3420, 3150, 2920, 2600, 1625, 1595, 1570, 1490, 1425, 1400, 1340, 1290, 1235, 1180, 1125, 1095, 1090, 1015, 830, 805 $cm^{-1}$;

NMR (methanol-$d_4$ solution): $\delta$=7.5-6.9 (7H, m), 2.30 (3H, s);

Mass: m/e=231 ($M^+$), 213, 199.

By the same procedure as described in Example 2, the amino compounds having the following physical characteristics were obtained from the corresponding nitro compounds.

(a) 2-Amino-4-(4-chlorophenylthio)phenol hydrochloride

Starting material: 800 mg of 4-(4-chlorophenylthio)-2-nitrophenol [prepared in Reference Example 4(b)];

Yield: 540 mg;

Melting point: 185-195°C (decomposed);

TLC (methylene chloride:methanol=10:1): Rf=0.61;

IR: $\nu$=3380, 3150, 3050, 2920, 2570, 1625, 1595, 1565, 1530, 1495, 1475,

1420, 1390, 1335, 1290, 1230, 1175, 1125, 1095, 1010, 815 $cm^{-1}$;

NMR (methanol-$d_4$ solution): δ=7.48 (1H, d), 7.39 (1H, dd), 7.3-7.2 (4H, m), 7.10 (1H, d);

Mass: m/e=251, 233, 219, 199, 171.

(b) 2-Amino-4-(4-hydroxyphenylthio)phenol

[The final process for preparing hydrochloric acid addition salt was not carried out.]

Starting material: 210 mg of 4-(4-hydroxyphenylthio)-2-nitrophenol [prepared in Reference Example 4(c)-(1)];

Yield: 30 mg;

Melting point: 161-165°C;

TLC (benzene:ethyl acetate=1:1): Rf=0.33;

IR: ν=3400 (broad), 1600, 1585, 1505 (shoulder), 1495, 1445, 1380, 1280, 1250 (broad), 1200, 1175, 1145, 1105, 1010, 930, 890, 820 $cm^{-1}$;

NMR (methanol-$d_4$ solution): δ=7.14 (2H, d), 6.73 (2H, d), 6.9-6.5 (3H, m);

Mass: m/e=233 ($M^+$), 215, 201, 177, 159.

(c) 2-Amino-4-(3-amino-4-hydroxyphenylthio)phenol

[The final process for preparing hydrochloric acid addition salt was not carried out.]

Starting material: 120 mg of 4-(4-hydroxy-3-nitrophenylthio)-2-nitrophenol [prepared in Reference Example 4(c)-(2)];

Yield: 57 mg;

Melting point: 198-202°C (decomposed);

TLC (benzene:ethyl acetate=1:1): Rf=0.24;

IR: ν=3400, 3320, 3100 (broad), 1600, 1500, 1440, 1380, 1285, 1200, 1155, 1120, 1080, 935, 880, 840, 820, 810 $cm^{-1}$;

NMR (methanol-$d_4$ + acetone-$d_6$ solution): δ=6.74 (2H, d), 6.62 (2H, d), 6.56 (2H, dd);

Mass: m/e=248 ($M^+$), 230, 202, 187, 141, 109, 96.

(d) 2-Amino-4-(2,4,6-trimethylphenylthio)phenol hydrochloride

Starting material: 600 mg of 4-(2,4,6-trimethylphenylthio)-2-nitrophenol [prepared in Reference Example 4(d)];

Yield: 400 mg;

Melting point: more than 190°C (decomposed);

TLC (methylene chloride:methanol=10:1): Rf=0.52;

IR: ν=3430, 3180, 2910, 2600, 1630, 1600, 1570, 1495, 1425, 1375, 1335, 1290, 1230, 1180, 1125, 1060, 1030, 850, 825 $cm^{-1}$;

NMR (methanol-$d_4$ solution): δ=7.02 (2H, s), 6.89 (3H, m), 2.37 (6H, s), 2.29 (3H, s);

Mass: m/e=259, 241, 227, 209, 151, 150, 109.

(e) 2-Amino-4-(4-tert-butylphenylthio)phenol hydrochloride

Starting material: 707 mg of 4-(4-tert-butylphenylthio)-2-nitrophenol [prepared in Reference Example 4(e)];

Yield: 615 mg;

Melting point: 169-175°C (decomposed);

TLC (methylene chloride:methanol=10:1): Rf=0.55;

IR: ν=3450, 2970, 2570, 1625, 1600, 1495, 1425, 1395, 1365, 1295, 1230, 1180, 1120, 1015, 820 $cm^{-1}$;

NMR (methanol-$d_4$ solution): δ=7.5-7.1 (6H, m), 7.02 (1H, d), 1.30 (9H, s);

Mass: m/e=273, 258, 230, 199, 171, 140, 115, 109, 96.

(f) 2-Amino-4-benzenesulfinylphenol

[The final process for preparing hydrochloric acid addition salt was not carried out.]

Starting material: 439 mg of 4-benzenesulfinyl-2-nitrophenol (prepared in Reference Example 5);

Yield: 290 mg;

Melting point: 152-154°C;

TLC (methylene chloride:methanol=10:1): Rf=0.46;

IR: ν=3480, 3370, 3060, 2935, 2760, 1620, 1580, 1510, 1445, 1395, 1290, 1220, 1140, 1085, 1065, 1010, 995, 810, 750, 690 $cm^{-1}$;

NMR (deuterochloroform + methanol-$d_4$ solution): δ=7.7-7.3 (5H, m), 7.0-6.7 (3H, m);

Mass: m/e=233 ($M^+$), 217, 199, 185, 171, 156, 124, 110.

(g) 2-Amino-4-(2-benzothiazolyl)phenol hydrochloride

Starting material: 160 mg of 4-(2-benzothiazolyl)-2-nitrophenol (prepared in Reference Example 8);

Yield: 108 mg;

Melting point: 196-200°C (decomposed);

TLC (methylene chloride:ethyl acetate=5:1): Rf=0.35;

IR: ν=3420, 2750, 2540, 1610, 1428, 1315, 1300 $cm^{-1}$;

NMR (dimethylsulfoxide-$d_6$ + deuterochloroform solution): δ= 8.11 (1H, d), 8.00 (2H, d), 7.90 (1H, dd), 7.56-7.38 (2H, m), 7.21 (1H, d);

Mass: m/e=242 ($M^+$), 213, 186.

(h) 2-Amino-4-phenylethynylphenol

[The final process for preparing hydrochloric acid addition salt was not carried out.]

Starting material: 41 mg of 2-nitro-4-phenylethynylphenol (prepared in Reference Example 11);

Yield: 14 mg;

TLC (methylene chloride:ethyl acetate=4:1): Rf=0.50;

IR: ν=3380, 3290, 2200, 1590, 1505, 1280, 1250, 900, 810, 750 $cm^{-1}$;

NMR (methanol-$d_4$ solution): δ=7.5-7.4 (2H, m), 7.4-7.25 (3H, m), 6.88 (1H, s), 6.77 (1H, d), 6.67 (1H, d);

Mass: m/e=209 ($M^+$).

Example 3

N-(3-Amino-4-hydroxyphenyl)benzamide hydrochloride

Under an atmosphere of hydrogen, to a solution of 96 mg of N-(4-hydroxy-3-nitrophenyl)benzamide (prepared in Reference Example 7) in 10 ml of ethanol was added 0.3 ml of chloroform and 50 mg of palladium-carbon (content: 10%). The mixture was stirred for 1.5 hours at room temperature. Catalyst was filtered off and the filtrate was concentrated under reduced pressure. The residue was dissolved in ethanol and thereto was added 0.2 ml of a concentrated hydrochloric acid. The mixture was treated with activated carbon and concentrated under reduced pressure to give 80 mg of the title compound having the following physical characteristics:

TLC (chloroform:methanol:ammonia water=60:6:1): Rf=0.17;

IR: ν=3400 (broad), 3060, 2900 (broad), 2580, 1640, 1545, 1510, 1450, 1440, 1295, 1210, 1110, 1075, 1025, 870, 820, 800 (shoulder), 710 $cm^{-1}$;

NMR (methanol-$d_4$ solution): δ=7.89 (3H, m), 7.47 (4H, m), 6.99 (1H, d);

Mass: m/e=228, 105, 77.

Example 4

5 g of 2-amino-4-phenylthiophenol hydrochloride, 200 mg of cellulose magnesium gluconate (disintegrator), 100 mg of magnesium stearate (libricating agent) and 4.7 g of crystal cellulose were mixed and punched out in a conventional method to obtain 100 tablets containing 50 mg of the active ingredient in one tablet.

Example 5

5 g of 2-amino-4-(4-methylphenylthio)phenol hydrochloride, 200 mg of cellulose magnesium gluconate (disintegrator), 100 mg of magnesium stearate (libricating agent) and 4.7 g of crystal cellulose were mixed and punched out in a conventional method to obtain 100 tablets containing 50 mg of the active ingredient in one tablet.

CLAIMS FOR THE DESIGNATED STATES:-
G.B, FR, DE, CH, LI, LU, BE, NL, IT, SE

1. A 2-aminophenol derivative of the general formula:

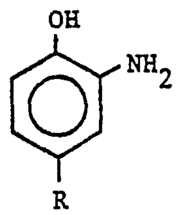

(I)

[wherein R represents a group of the general formula:

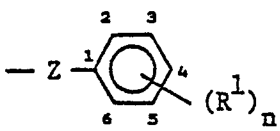

(IIa)

(in which Z represents a sulfur atom or a sulfinyl group and $(R^1)_n$ represents that:

(i) n is an integer of 1, 2 or 3 and $R^1$, which may be the same or different, is a hydrogen atom, a straight- or branched-chain alkyl group containing from 1 to 6 carbon atoms or a halogen atom;

(ii) n is an integer of 1, 2 or 3 and $R^1$ is a hydroxy group; or

(iii) n is an integer of 2 and two $R^1$s are a hydroxy group and an amino group, a hydroxy group and a straight- or branched-chain alkyl group containing from 1 to 6 carbon atoms, or a hydroxy group and a halogen atom, such groups being at o-position or p-position

to each other), or

R represents a group of the general formula:

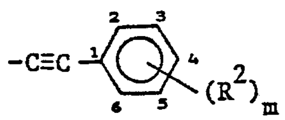

(IIb)

(in which m is an integer of 1 or 2 and $R^2$, which may be the same or different, represents a hydrogen atom, a straight- or branched-chain alkyl group containing from 1 to 6 carbon atoms or a halogen atom), or R represents a group of the general formula:

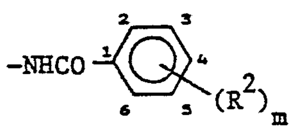

(IIc)

(in which the various symbols are as hereinbefore defined), or R represents a group of the general formula:

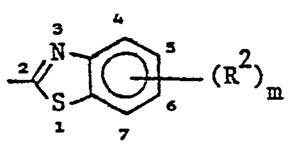

(IId)

(in which the various symbols are as hereinbefore defined)], or a pharmaceutically-acceptable acid addition salt thereof.

2. A compound according to claim 1, wherein R represents a group of the general formula:

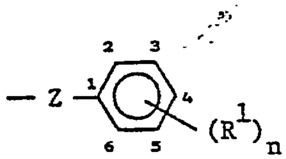

(IIa)

(wherein the various symbols are as defined in claim 1).

3. A compound according to claim 2, wherein Z, in the general formula (IIa), represents a sulfur atom.

4. A compound according to claim 2, wherein Z, in the general formula (IIa), represents a sulfinyl group.

5. A compound according to claim 3 or 4, wherein $(R^1)_n$, in the general formula (IIa), represents that:

(i) n is an integer of 1 and $R^1$ is a hydrogen atom, a straight- or branched-chain alkyl group containing from 1 to 6 carbon atoms or a halogen atom, or n is an integer of 3 and $R^1$, which may be the same or different, is a straight- or branched-chain alkyl group containing from 1 to 6 carbon atoms;

(ii) n is an integer of 1 and $R^1$ is a hydroxy group; or

(iii) n is an integer of 2 and two $R^1$s are a hydroxy group and an amino group, such groups being at o-position or p-position to each other.

6. A compound according to claim 5, wherein $(R^1)_n$, in the general formula (IIa), is attached:

(a) to the 4th position, or to the 2nd, 4th and 6th positions of the benzene ring when $(R^1)_n$ represents the case (i) in claim 5,

(b) to the 4th position of the benzene ring when $(R^1)_n$ represents the case (ii) in claim 5, or

(c) to the 3rd and 4th positions of the benzene ring when $(R^1)_n$ represents the case (iii) in claim 5.

7. A compound according to claim 6, which is 2-amino-4-phenylthiophenol or its hydrochloride.

8. A compound according to claim 6, which is 2-amino-4-(4-methylphenylthio)phenol or its hydrochloride.

9. A compound according to claim 6, which is 2-amino-4-(4-tert-butylphenylthio)phenol or its hydrochloride.

10. A compound according to claim 6, which is 2-amino-4-(2,4,6-trimethylphenylthio)phenol or its hydrochloride.

11. A compound according to claim 6, which is 2-amino-4-(4-chlorophenylthio)phenol or its hydrochloride.

12. A compound according to claim 6, which is 2-amino-4-(4-hydroxyphenylthio)phenol or its hydrochloride.

13. A compound according to claim 6, which is 2-amino-4-(3-amino-4-hydroxyphenylthio)phenol or its hydrochloride.

14. A compound according to claim 6, which is 2-amino-4-benzenesulfinylphenol or its hydrochloride.

15. A compound according to claim 1, wherein R represents a group of the general formula:

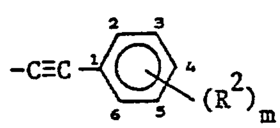

(IIb)

(wherein the various symbols are as defined in claim 1).

16. A compound according to claim 1, wherein R represents a group of the general formula:

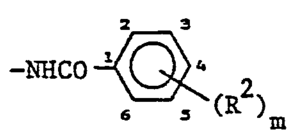

(IIc)

(wherein the various symbols are as defined in claim 1).

17. A compound according to claim 1, wherein R represents a group of the general formula:

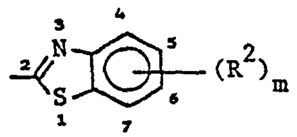

(IId)

(wherein the various symbols are as defined in claim 1).

18. A compound according to claim 15, 16 or 17, wherein $(R^2)_m$, in the general formula (IIb), (IIc) or (IId), represents that m is an integer of 1 and $R^2$ is a hydrogen atom, a straight- or branched-chain alkyl group containing from 1 to 6 carbon atoms or a halogen atom.

19. A compound according to claim 18, wherein $R^2$, in the general formula (IIb) or (IIc), is attached to the 4th position of the benzene ring, or $R^2$, in the general formula (IId), is attached to the 5th or 6th position of the benzothiazole ring.

20. A compound according to claim 19, which is 2-amino-4-phenylethynylphenol or its hydrochloride.

21. A compound according to claim 19, which is N-(3-amino-4-hydroxyphenyl)benzamide or its hydrochloride.

22. A compound according to claim 19, which is 2-amino-4-(2-benzothiazolyl)phenol or its hydrochloride.

23. A process for the preparation of a 2-aminophenol derivative as claimed in claim 1 and conforming to the general formula:

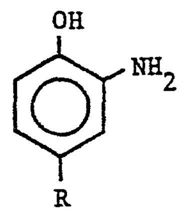

(I)

(wherein the various symbols are as defined in claim 1) or a pharmaceutically-acceptable acid addition salt thereof, which comprises selectively reducing the nitro group (and $R^3$, when one of $R^3$ represents a nitro group) of a compound of the general formula:

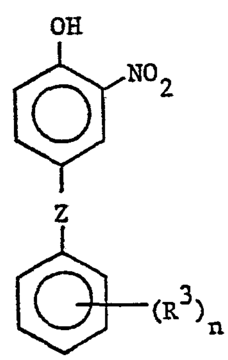

(IIIa),

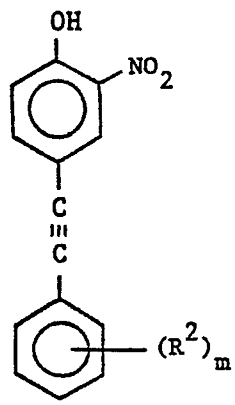

(IIIb),

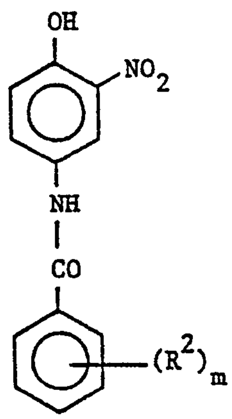

(IIIc)

or

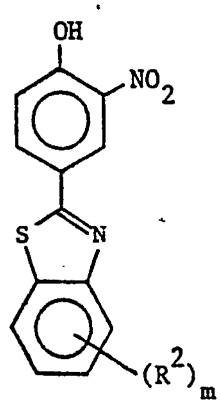

(IIId)

(wherein $(R^3)_n$ represents that:

(i) n is an integer of 1, 2 or 3 and $R^3$, which may be the same or different, is a hydrogen atom, a straight- or branched-chain alkyl group containing from 1 to 6 carbon atoms or a halogen atom;

(ii) n is an integer of 1, 2 or 3 and $R^3$ is a hydroxy group; or

(iii) n is an integer of 2 and two $R^3$s are a hydroxy group and a nitro group, a hydroxy group and a straight- or branched-chain alkyl group containing from 1 to 6 carbon atoms, or a hydroxy group and a halogen atom, such groups being at o-position or p-position to each other,

and the other symbols are as hereinbefore defined) to convert to amino group(s) to obtain a compound of the general formula:

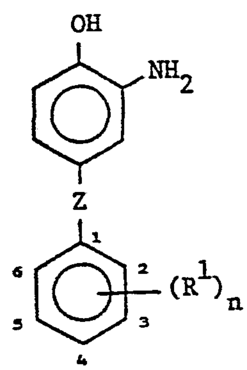

(Ia),

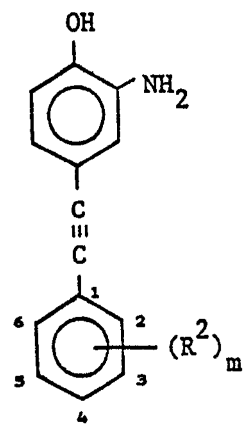

(Ib),

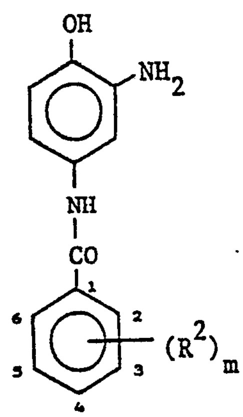

(Ic)

or

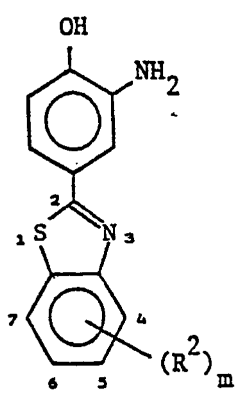

(Id)

(wherein the various symbols are as hereinbefore defined), respectively, or converting the 2-aminophenol derivative of the general formula (I) obtained into a pharmaceutically-acceptable acid addition salt thereof by methods known per se.

24. A pharmaceutical composition for the prevention and the treatment of allergic tracheal and bronchial diseases or allergic lung diseases, allergic shocks or various allergic inflammations, or for the prevention and the treatment of various inflammations induced by prostaglandins, which comprises, as active ingredient, an effective amount of at least one compound of the general formula (I) depicted in claim 1, wherein the various symbols are as defined in claim 1, or a pharmaceutically-acceptable acid addition salt thereof, together with a pharmaceutical carrier or coating.

CLAIMS FOR THE DESIGNATED STATE: AU

1. A process for the preparation of a 2-aminophenol derivative of the general formula:

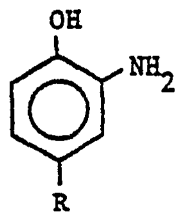

(I)

[wherein R represents a group of the general formula:

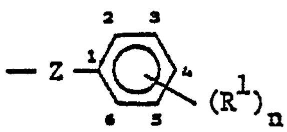

(IIa)

(in which Z represents a sulfur atom or a sulfinyl group and $(R^1)_n$ represents that:

(i) n is an integer of 1, 2 or 3 and $R^1$, which may be the same or different, is a hydrogen atom, a straight- or branched-chain alkyl group containing from 1 to 6 carbon atoms or a halogen atom;

(ii) n is an integer of 1, 2 or 3 and $R^1$ is a hydroxy group; or

(iii) n is an integer of 2 and two $R^1$s are a hydroxy group and an amino group, a hydroxy group and a straight- or branched-chain alkyl group containing from 1 to 6 carbon atoms, or a hydroxy group and a halogen atom, such groups being at o-position or p-position

to each other), or

R represents a group of the general formula:

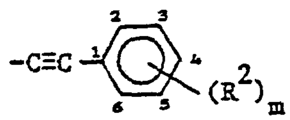

(IIb)

(in which m is an integer of 1 or 2 and $R^2$, which may be the same or different, represents a hydrogen atom, a straight- or branched-chain alkyl group containing from 1 to 6 carbon atoms or a halogen atom), or

R represents a group of the general formula:

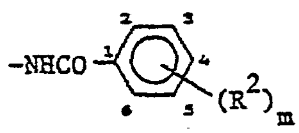

(IIc)

(in which the various symbols are as hereinbefore defined), or

R represents a group of the general formula:

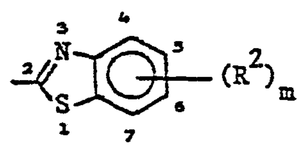

(IId)

(in which the various symbols are as hereinbefore defined)], or a pharmaceutically-acceptable acid addition salt thereof, which comprises selectively reducing the nitro group (and $R^3$, when one of $R^3$ represents a nitro group) of a compound of the general formula:

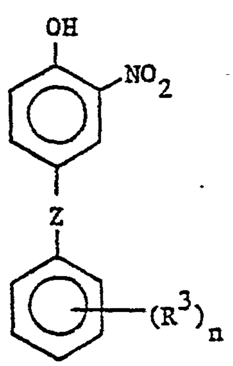

(IIIa),

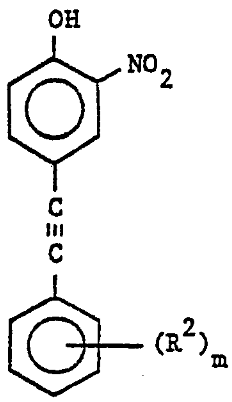

(IIIb),

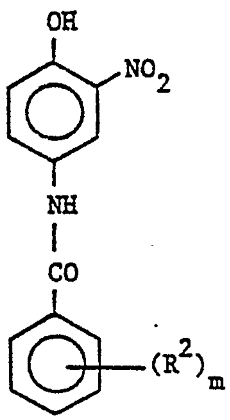

(IIIc)

or

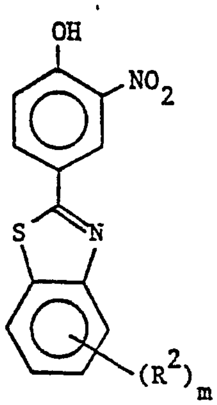

(IIId)

(wherein $(R^3)_n$ represents that:

(i) n is an integer of 1, 2 or 3 and $R^3$, which may be the same or different, is a hydrogen atom, a straight- or branched-chain alkyl group containing from 1 to 6 carbon atoms or a halogen atom;

(ii) n is an integer of 1, 2 or 3 and $R^3$ is a hydroxy group; or

(iii) n is an integer of 2 and two $R^3$s are a hydroxy group and a nitro group, a hydroxy group and a straight- or branched-chain alkyl group containing from 1 to 6 carbon atoms, or a hydroxy group and a halogen atom, such groups being at o-position or p-position to each other,

and the other symbols are as hereinbefore defined) to convert to amino group(s) to obtain a compound of the general formula:

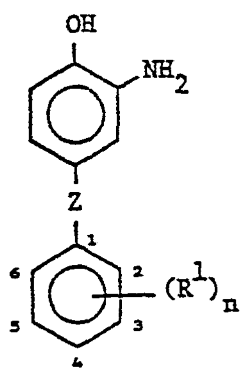

(Ia),

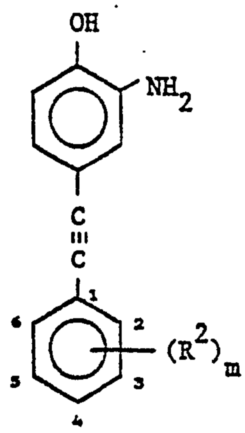

(Ib),

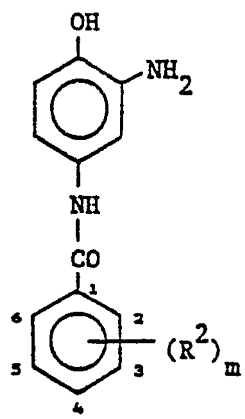

(Ic)

or

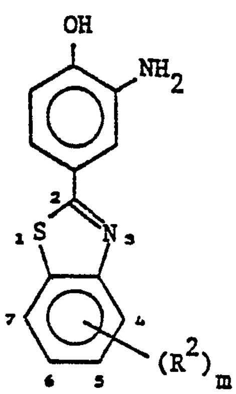

(Id)

(wherein the various symbols are as hereinbefore defined), respectively, or converting the 2-aminophenol derivative of the general formula (I) obtained into a pharmaceutically-acceptable acid addition salt thereof by methods known per se.

2. A process according to claim 1, wherein R represents a group of the general formula:

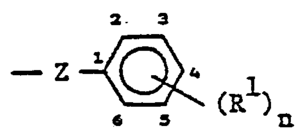

(IIa)

(wherein the various symbols are as defined in claim 1).

3. A process according to claim 2, wherein Z, in the general formula (IIa), represents a sulfur atom.

4. A process according to claim 2, wherein Z, in the general formula (IIa), represents a sulfinyl group.

5. A process according to claim 3 or 4, wherein $(R^1)_n$, in the general formula (IIa), represents that:

(i) n is an integer of 1 and $R^1$ is a hydrogen atom, a straight- or branched-chain alkyl group containing from 1 to 6 carbon atoms or a halogen atom, or n is an integer of 3 and $R^1$, which may be the same or different, is a straight- or branched-chain alkyl group containing from 1 to 6 carbon atoms;

(ii) n is an integer of 1 and $R^1$ is a hydroxy group; or

(iii) n is an integer of 2 and two $R^1$s are a hydroxy group and an amino group, such groups being at o-position or p-position to each other.

6. A process according to claim 5, wherein $(R^1)_n$, in the general formula (IIa), is attached:

(a) to the 4th position, or to the 2nd, 4th and 6th positions of the benzene ring when $(R^1)_n$ represents the case (i) in claim 5,

(b) to the 4th positions of the benzene ring when $(R^1)_n$ represents the case (ii) in claim 5, or

(c) to the 3rd and 4th positions of the benzene ring when $(R^1)_n$ represents the case (iii) in claim 5.

7. A process according to claim 6, in which the compound is 2-amino-4-phenylthiophenol or its hydrochloride.

8. A process according to claim 6, in which the compound is 2-amino-4-(4-methylphenylthio)phenol or its hydrochloride.

9. A process according to claim 6, in which the compound is 2-amino-4-(4-tert-butylphenylthio)phenol or its hydrochloride.

10. A process according to claim 6, in which the compound is 2-amino-4-(2,4,6-trimethylphenylthio)phenol or its hydrochloride.

11. A process according to claim 6, in which the compound is 2-amino-4-(4-chlorophenylthio)phenol or its hydrochloride.

12. A process according to claim 6, in which the compound is 2-amino-4-(4-hydroxyphenylthio)phenol or its hydrochloride.

13. A process according to claim 6, in which the compound is 2-amino-4-(3-amino-4-hydroxyphenylthio)phenol or its hydrochloride.

14. A process according to claim 6, in which the compound is 2-amino-4-benzenesulfinylphenol or its hydrochloride.

15. A process according to claim 1, wherein R represents a group of the general formula:

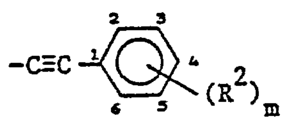

(IIb)

(wherein the various symbols are as defined in claim 1).

16. A process according to claim 1, wherein R represents a group of the general formula:

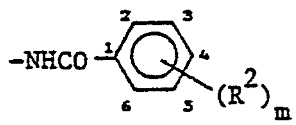

(IIc)

(wherein the various symbols are as defined in claim 1).

17. A process according to claim 1, wherein R represents a group of the general formula:

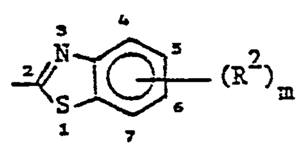

(IId)

(wherein the various symbols are as defined in claim 1).

18. A process according to claim 15, 16 or 17, wherein $(R^2)_m$, in the general formula (IIb), (IIc) or (IId), represents that m is an integer of 1 and $R^2$ is a hydrogen atom, a straight- or branched-chain alkyl group containing from 1 to 6 carbon atoms or a halogen atom.

19. A process according to claim 18, wherein $R^2$, in the general formula (IIb) or (IIc), is attached to the 4th position of the benzene ring, or $R^2$, in the general formula (IId), is attached to the 5th or 6th position of the benzothiazole ring.

20. A process according to claim 19, in which the compound is 2-amino-4-phenylethynylphenol or its hydrochloride.

21. A process according to claim 19, in which the compound is N-(3-amino-4-hydroxyphenyl)benzamide or its hydrochloride.

22. A process according to claim 19, in which the compound is 2-amino-4-(2-benzothiazolyl)phenol or its hydrochloride.